# EUROPEAN PATENT APPLICATION

(11) **EP 4 354 139 A1**
(43) Date of publication of application: **17.04.2024**
(21) Application number: 22382976.3
(22) Date of filing: 13.10.2022
(51) Int. Cl.: G01N 33/50, G01N 33/533, G01N 33/58, C12Q 1/6841

(54) **NON-INVASIVE METHOD OF CELL IMAGING**

(71) Applicant: Fundación Carlos Simón para la Investigación en Salud de la Mujer, 46012 Valencia (ES); The Trustees of the University of Pennsylvania, Philadelphia, PA 19104 (US)
(72) Inventor: PLACHTA, Nicolas, Philadelphia, 19104 (US); SIMÓN, Carlos, 46012 Valencia (ES); BISSIERE, Stephanie, Philadelphia, 19104 (US)
(74) Representative: Hoffmann Eitle

(57) **Abstract**

The present invention relates to a method for obtaining one or more fluorescent images of one or more cells, the method comprising the steps of incubating the one or more cells with a first probe for fluorescence imaging of nuclear DNA and with a second probe for fluorescence imaging of actin and visualizing of the one or more cells stained with both probes by fluorescence microscopy. The method is particularly useful for monitoring embryo development and for diagnosing aneuploidy.

## Description

### TECHNICAL FIELD

The present invention relates to the field of microscopy, especially embryo imaging. The invention provides methods to accurately visualize central events occurring in an embryo, particularly during the preimplantation development of a mammalian embryo, as well as methods of selecting or grading embryos according to their development and genetic quality.

### BACKGROUND ART

Following fertilization, the human embryo develops as a self-contained structure and undergoes a series of cleavage divisions culminating with the formation of the blastocyst. This preimplantation stage of development is critical to establish a successful pregnancy but has thus far been studied predominantly in the mouse embryo.

Classically analysed using histology in fixed specimens, advances in genetic tools for fluorescently labeling cells and live-imaging has made it possible to non-invasively visualize the dynamic course of events driving early mouse development. Microscopy thus became a central tool in developmental biology, both for determining the normal course of developmental events and for investigating the consequences of experimental perturbations. Embryos have been visualized to reveal gross morphological features, or after sectioning and histological staining, to provide cellular resolution. Dynamics and the sequence of events are usually inferred by analyzing multiple sequentially staged embryos. The ability to observe a single specimen continuously and at cellular resolution holds great promise and represents the evolution of classical histology into digitized multidimensional information documenting actual developmental progression.

Certain applications, for example, determining gross tissue organization, are better served by imaging of large samples (e.g., whole embryos) at lower resolution, while others require investigations at the subcellular level. As a consequence, imaging modalities such as optical projection tomography (OPT), optical coherence tomography (OCT), and magnetic resonance imaging (MRI) have been developed to provide insight into embryogenesis and the global organization of structures, while others such as of laser-scanning microscopy (LSM) have been used to study at a cellular or subcellular resolution. Recent advances in microscopy techniques for data acquisition as well as in computational methods for data processing in combination with the ever-increasing repertoire of reporter strains expressing spectrally-distinct genetically-encoded fluorescent proteins provide powerful tools for the generation of 3D time-lapse information. Moreover, such data promise to provide insight not only to the field of developmental biology but also beyond, by furthering our understanding of homeostasis and disease progression.

Fluorescent molecules are thus routinely used as vital reporters to label tissues, cells, cellular organelles, or proteins of interest. In doing so, they provide contrasting agents enabling the acquisition of high-resolution quantitative image data. With the advent of more accessible and sophisticated imaging technologies and increased abundance of fluorescent molecules with different spectral characteristics, the dynamic processes taking place in situ in living embryos can now be probed.

For instance, using live mouse embryo imaging, it has been demonstrated that cells extend long E-cadherin-dependent filopodia on to neighbouring cells, which control the cell shape changes necessary for compaction (https://doi.org/10.1038/ncb2875). In another study (https://doi.org/10.1016/j.devcel.2015.07.004), high-resolution membrane segmentation was used to show that inner cells originate primarily by cell internalization events, which were identified as apical constriction by combining live imaging, computational analysis, and molecular manipulations. Zenker et al. (https://doi.org/10.1016/j.cell.2018.02.035) used live imaging of mouse embryos to unveil the actin-zippering mechanism driving this embryo sealing. Live embryo imaging was also used to study the mechanism by which cell-to-cell heterogeneities that appear before the segregation of the trophectoderm and the inner cell mass influence lineage fate (https://doi.org/10.1038/s41586-020-2647-4).

Similar approaches would prove powerful to study human preimplantation development. However, most legislations pose restrictions on the use of genetic manipulation and microinjection for human preimplantation research. Furthermore, human embryos donated for research are typically obtained at the blastocyst stage, which contains hundreds of cells. This is incompatible with microinjection of mRNA or DNA, which is performed in the 1-cell embryo to express fluorescent proteins for live imaging studies.

Thus, establishing approaches that combine non-invasive labeling of cells with live-imaging remains an open challenge to uncover human preimplantation development.

### SUMMARY OF INVENTION

A first aspect relates to an in vitro method of obtaining one or more fluorescent images of one or more isolated cells, the method comprising the steps of:
(a) incubating the one or more isolated cells with a first probe for fluorescence imaging of nuclear DNA, wherein the first probe comprises a molecule capable of binding to nuclear DNA conjugated to a first fluorophore, and wherein the incubation produces a stain indicative of the presence of nuclear DNA,
(b) prior to, simultaneously to, or subsequently to step (a), incubating the one or more isolated cells with a second probe for fluorescence imaging of actin, wherein the second probe comprises a molecule capable of binding to actin conjugated to a second fluorophore, and wherein the incubation produces a stain indicative of the presence of actin, and
(c) providing one or more images of the one or more isolated cells that have been stained with both the first and the second probe, said one or more images being obtained by fluorescence microscopy,
wherein the first and second probes are cell permeable, wherein one of the fluorophores, preferably the first fluorophore, has a maximum absorbance wavelength (A_{λ}) of 652 nm and a maximum fluorescence wavelength (F_{λ}) of 674 nm and the other fluorophore, preferably the second fluorophore, has a maximum absorbance wavelength (A_{λ}) of 555 nm and a maximum fluorescence wavelength (F_{λ}) of 580 nm, and wherein the in vitro method does not involve any damage or destruction of the one or more cells to which the method is applied, i.e., it is non-destructive. Preferably, the method is also non-invasive. Most preferably, the first and second probes are cell permeable and non-invasive, thereby not affecting nor damaging the one or more cells, most preferably when said one or more cells are comprised in an embryo.

Preferably, the probe for fluorescence imaging of actin specifically labels F-actin. Preferably, the molecule capable of binding to nuclear DNA is a Bisbenzimide compound or a Hoeschst stain. Preferably, the molecule capable of binding to actin is a Jasplakinolide molecule.

Preferably, the one or more isolated cells are comprised in a mammalian embryo, wherein said mammalian embryo is cultured under conditions to promote survival, growth and/or development, and wherein the one or more images provided in step (c) are analysed to determine whether the embryo presents developmental abnormalities as compared to control embryos.

Preferably, the one or more isolated cells are comprised in a mammalian embryo, wherein said mammalian embryo is cultured under conditions to promote survival, growth and/or development, and wherein the method comprises a step (d) of visualizing on the one or more images provided by fluorescence microscopy the labelled nuclear DNA of the one or more cells and determining the presence or absence of nuclear DNA loss events in said cells, said events being characterized by the presence of labelled nuclear DNA protruding outwards from the nucleus into the cytoplasm of said one or more cells.

Preferably, step (c) is performed for two or more time intervals thereby providing time-lapse images of the mammalian embryo, preferably wherein said time intervals are performed during the morphological events in the embryo development that take place when the embryo is in the blastocyst stage, preferably when the trophectoderm layer becomes constrained during cavity expansion in the blastocyst.

Preferably, the mammalian embryo is diagnosed with aneuploidy if nuclear DNA loss events are determined in at least one of its cells comprised in the embryo.

Preferably, the mammalian embryo is a human embryo.

A further aspect relates to the in vitro use of a first probe in combination with a second probe for obtaining a fluorescent image of one or more isolated cells, wherein the first probe comprises a molecule capable of binding to nuclear DNA conjugated to a first fluorophore, wherein the second probe comprises a molecule capable of binding to actin conjugated to a second fluorophore, wherein one of the fluorophores, preferably the first fluorophore, has a maximum absorbance wavelength (A_{λ}) of 652 nm and a maximum fluorescence wavelength (F_{λ}) of 674 nm and the other fluorophore, preferably the second fluorophore, has a maximum absorbance wavelength (A_{λ}) of 555 nm and a maximum fluorescence wavelength (F_{λ}) of 580 nm.

A further aspect relates to the vitro use of a first probe in combination with a second probe for determining whether one or more cells that are undergoing cell division are losing or will lose part of their nuclear DNA, wherein the first probe comprises a molecule capable of binding to nuclear DNA conjugated to a first fluorophore, wherein the second probe comprises a molecule capable of binding to actin conjugated to a second fluorophore, wherein one of the fluorophores, preferably the first fluorophore, has a maximum absorbance wavelength (A_{λ}) of 652 nm and a maximum fluorescence wavelength (F_{λ}) of 674 nm and the other fluorophore, preferably the second fluorophore, has a maximum absorbance wavelength (A_{λ}) of 555 nm and a maximum fluorescence wavelength (F_{λ}) of 580 nm.

A further aspect relates to a computer implemented method for carrying out the method or the uses defined in any of the previous aspects.

A further aspect relates to computer program product comprising instructions such that when processed by a processor, configures it to carry out the method or the uses defined in any of the previous aspects.

A further aspect relates to a kit suitable for obtaining one or more fluorescent images of one or more isolated cells, the kit comprising a first and a second probe as defined in the previous aspects.

A further aspect relates to the uses of the kit in the method or the uses defined in any of the previous aspects.

### BRIEF DESCRIPTION OF DRAWINGS

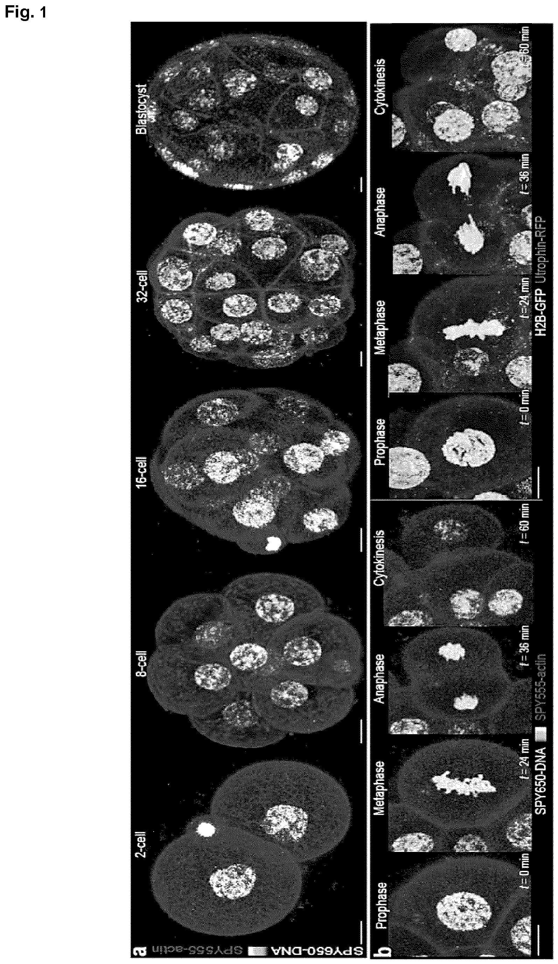

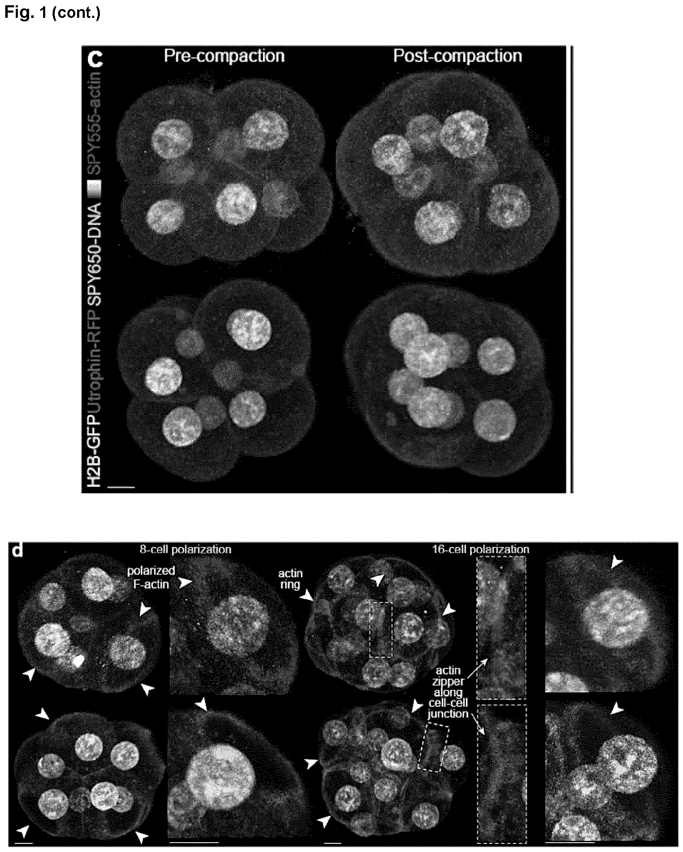

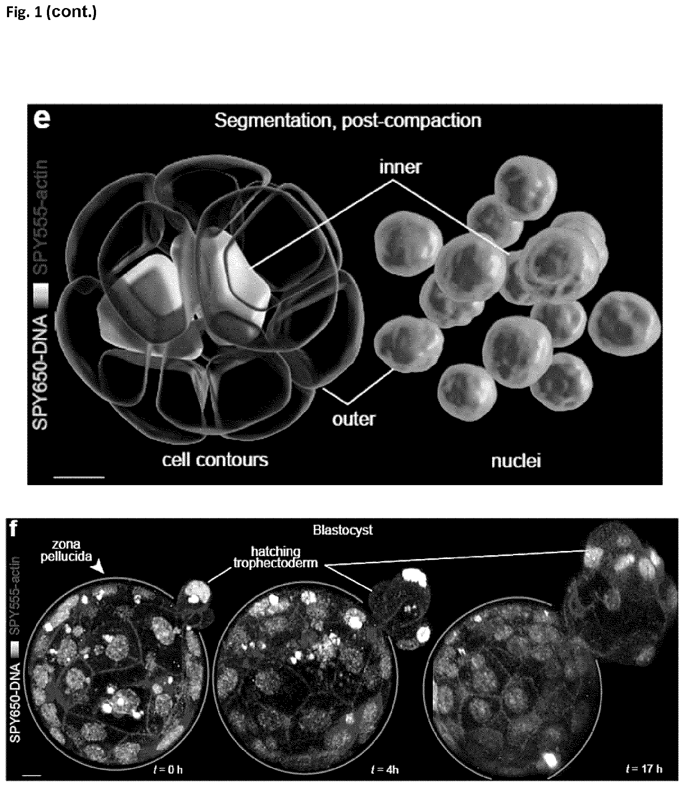

**Fig. 1: Fig. 1****. Imaging live mouse embryos with fluorescent dyes can bypass the need for genetic manipulation or mRNA injection. a**, Live-imaging of mouse embryos labeled with SPY650-DNA and SPY-555-actin at various developmental stages. **b-f**, The combination of SPY650-DNA and SPY-555-actin allows visualization of key events characterizing preimplantation development including the main phases of mitosis (**b**), embryo compaction (**c**), formation of F-actin-rich apical domains (at the 8-cell stage) and of F-actin rings that undergo zippering along cell-cell junctions (at the 16-cell stage) (**d**), visualization of the first inner cells of the embryo that will form the ICM, which can be computationally segmented within the 16-cell embryo (**e**), and blastocyst hatching from the zona pelucida (**f**). In **b** and **d**, embryos microinjected with mRNA for H2B-GFP and Utr-GFP are shown for comparison with the SPY650-DNA and SPY-555-actin approach. Scale bars, 10 µm.

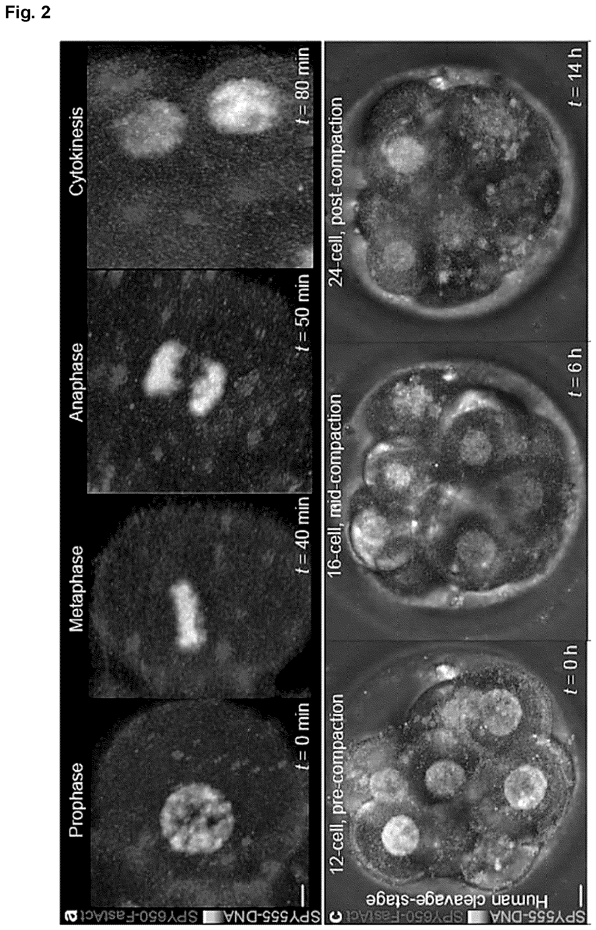

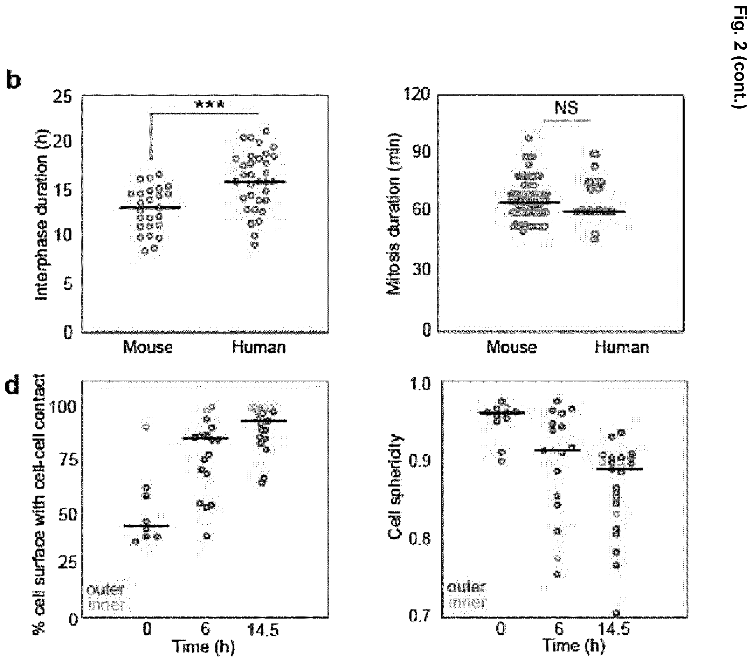

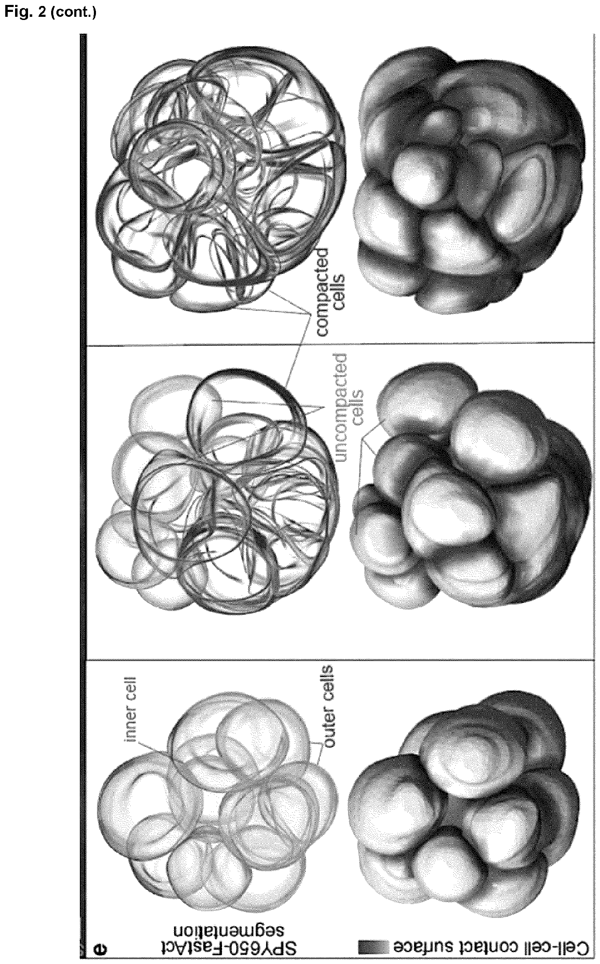

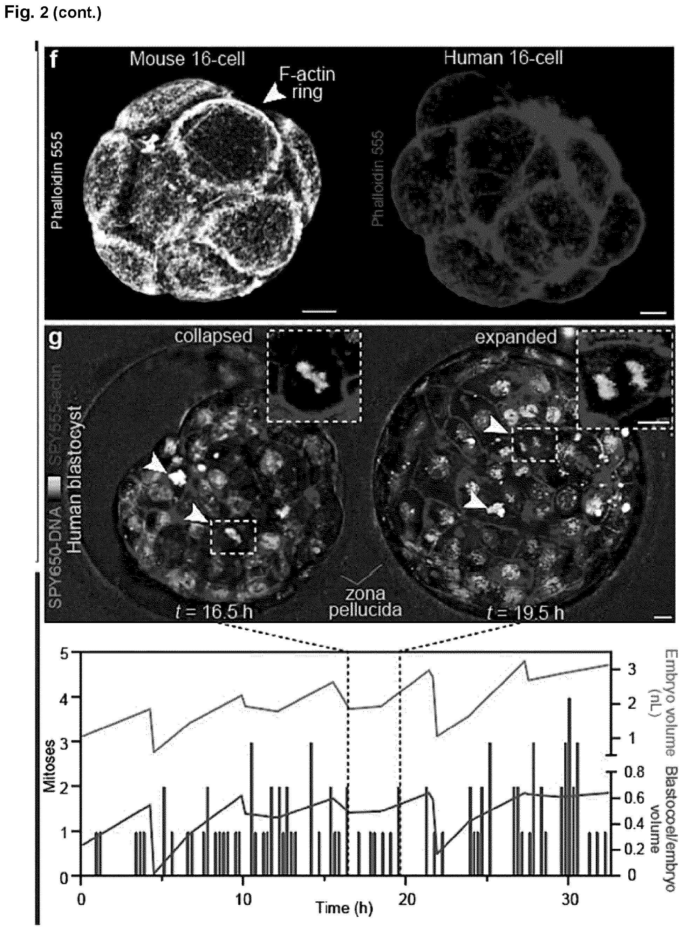

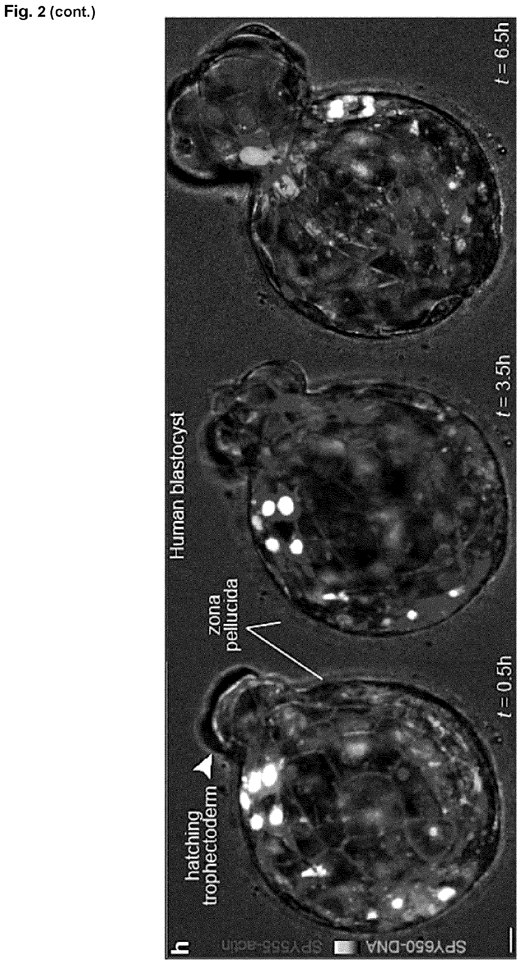

**Fig. 2: Fig. 2****. Non-invasive imaging reveals cell dynamics underlying human preimplantation development. a**, Live-imaging of cleavage stage human embryos labeled with SPY650-DNA and SPY555-actin allows visualization of the main phases of mitosis. **b**, Duration of interphase and mitosis in live mouse and human embryos stained with SPY650-DNA and SPY555-actin, ***p< 0.001, NS = not significant by student's t-test, *n*=cells. **c**, Live-imaging of cleavage-stage human embryos stained with SPY650-DNA and SPY-555-actin undergoing compaction. **d**, Quantitative analysis of changes in cell-cell contact and cell sphericity as proxies for compaction. **e**, Computational segmentation of embryos shown in c enables visualization of changes in cell morphology during compaction. Note that compaction occurs in an asynchronous manner and that an inner cell becomes completely enclosed by its neighbors during the compaction process, **f**, Unlike the mouse embryo, the human embryo does not display well-defined F-actin domains at the apical cell pole. **g**, Live-imaging of human blastocysts show the dynamics of embryo collapse and expansion. Note the lack of correlation to cell divisions. Images of the same blastocyst stained with SPY650-DNA and SPY555-actin after 16 and 19.5 h of imaging allowing identification of mitotic figures (arrowheads, insets). **h**, Selected frames of live human embryo undergoing hatching from the zona pelucida. Scale bars, 10 µm.

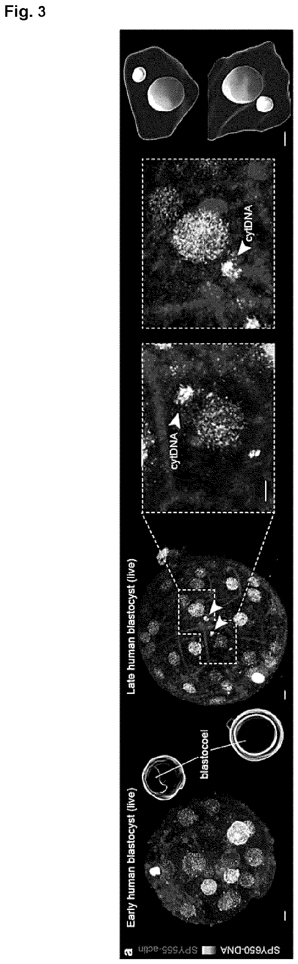

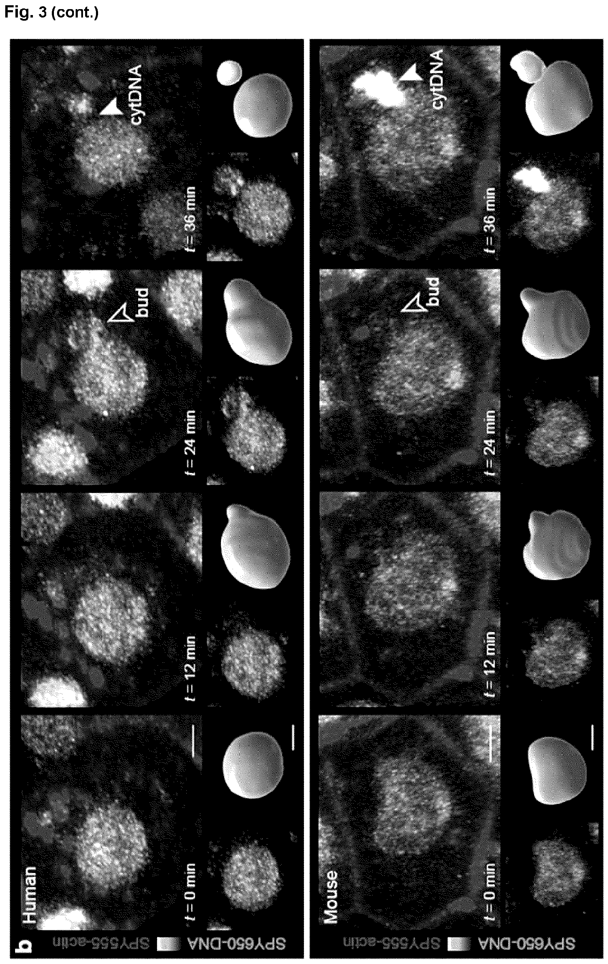

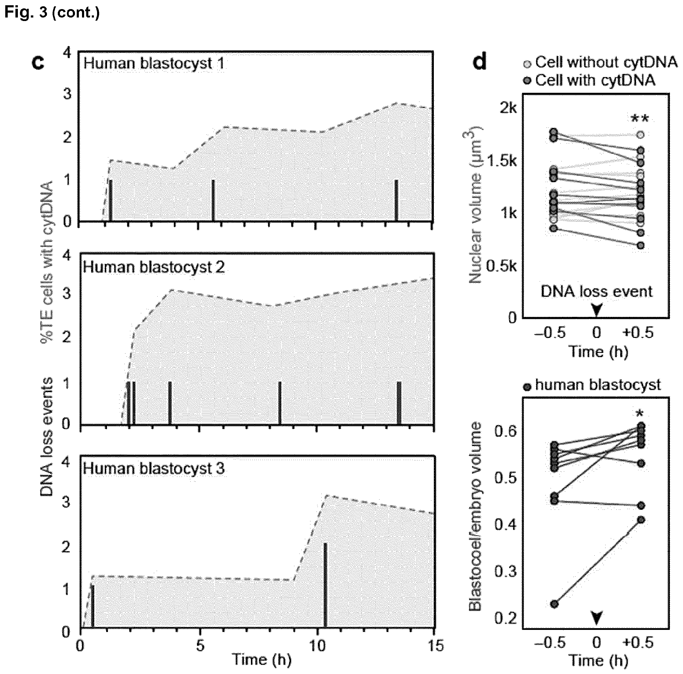

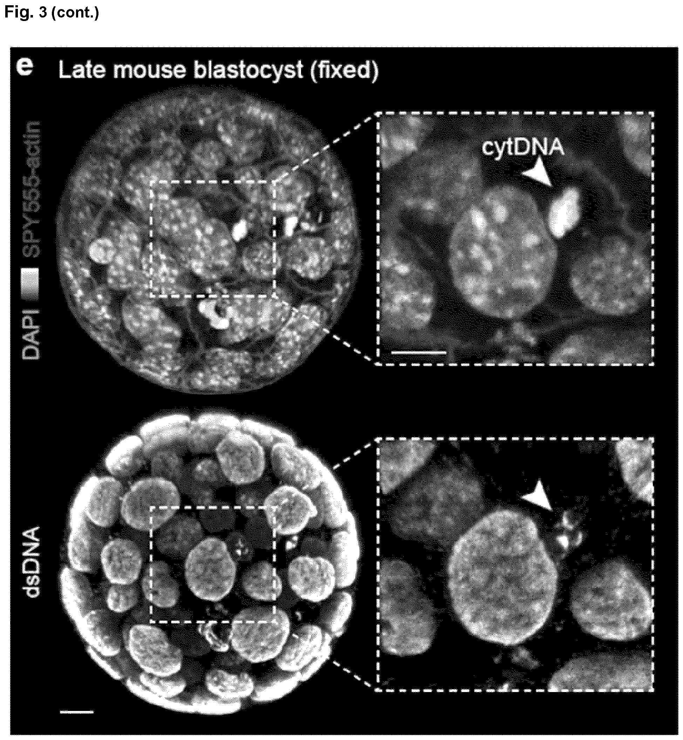

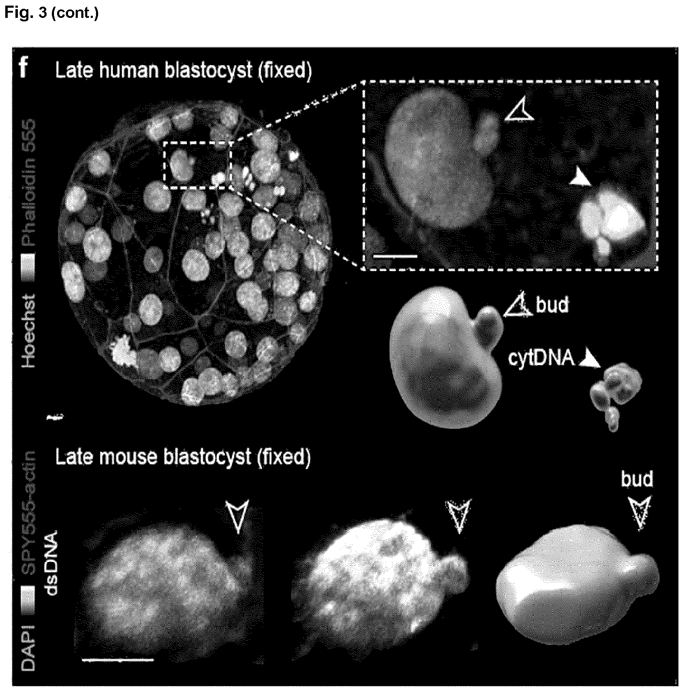

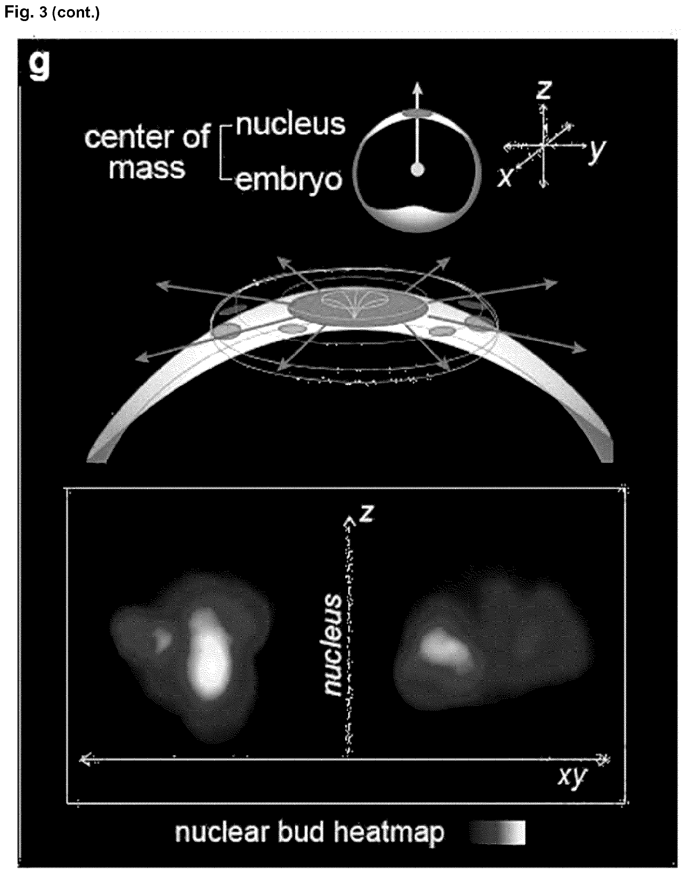

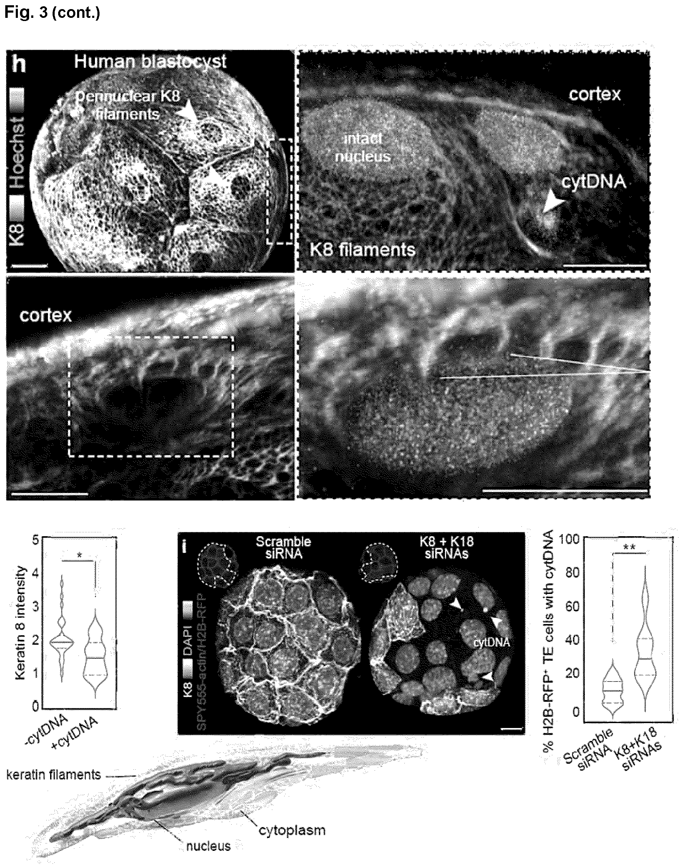

**Fig. 3****: Identification of DNA loss from cell nuclei.** a, Images of live human blastocysts uncover prominent SPY650-DNA-labeled structures within the cytoplasm in advanced-stage blastocysts. Smaller insets show segmented embryos highlighting the increase in blastocoel volume at the advanced stage (left panels), and the segmented SPY650-DNA and SPY555-actin signals highlight cytDNA structures contained within the cytoplasm (right panels). b, Live-imaging in human and mouse blastocysts demonstrate the appearance of nuclear buds and cytDNA in real time. Lower panels show SPY650-DNA signal segmented. c, Quantification of nuclear DNA loss events producing cytDNA in three embryos over time. d, Analysis of nuclear and blastocoel volumes pre- and post-DNA loss (arrowhead indicates the time of DNA loss). e and f, Detection of cytDNA structures in advanced-stage mouse and human blastocysts fixed and stained with DAPI or Hoechst, and with antibodies against double-stranded DNA. g, Analysis of nuclear bud position relative to the embryo's center of mass shows that buds are produced along the xy axis (i.e., orthogonal to the apical-basal axis of the cell). h, Human blastocyst showing keratin network labeled with K8 antibodies around most trophectoderm cell nuclei. Higher magnification images and computer segmentation of the keratin network highlight the cage-like organization of keratin filaments surrounding the cell nucleus. Analysis of K8 fluorescence intensity shows lower K8 levels in cells with cytDNA structures (+cytDNA). Graph shows analysis of cytDNA structures relative to keratin levels, probed via K8 immunofluorescence. i, siRNAs for K8+K18 injected in half of the embryo disrupt the keratin network (labeled via K8 immunofluorescence) and cause an increased number of cells with cytDNA. Scale bars, 10 µm.

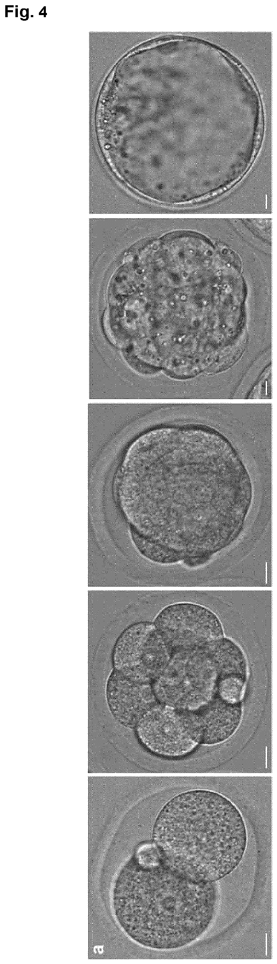

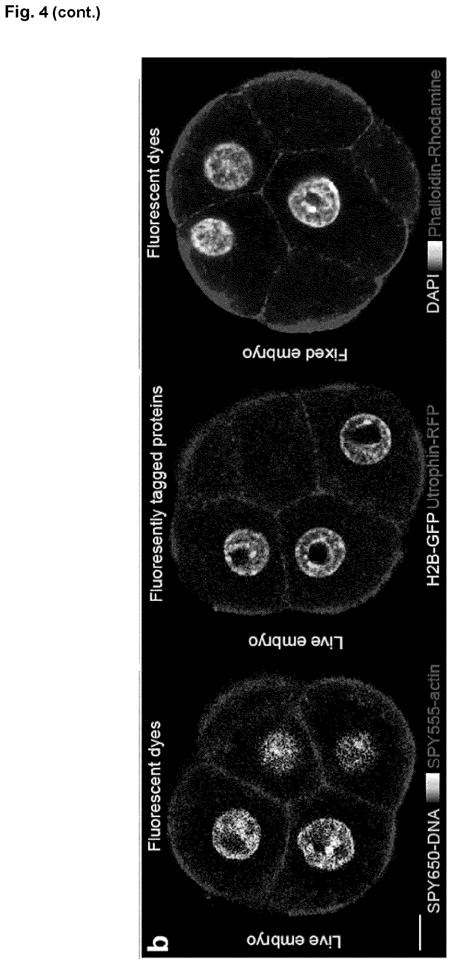

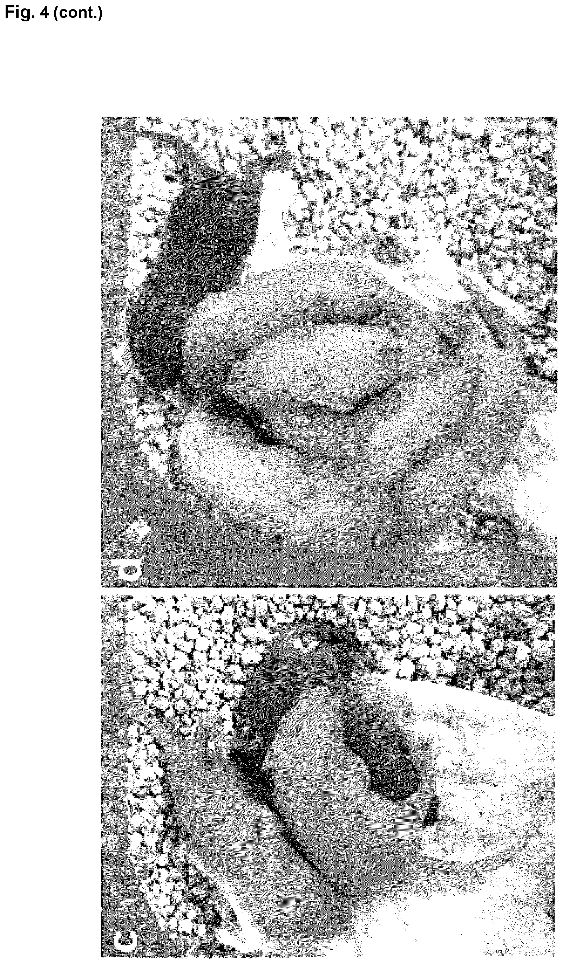

**Fig. 4****: Additional characterization of fluorescent dyes.** a, Brightfield images corresponding to the embryos shown in Fig. 1a. b, Incubation of live mouse embryos with SPY650-DNA and SPY555-actin followed by washing of the dyes produces similar labeling pattern to that found in live embryos microinjected with mRNA for H2B-GFP and Utr-GFP, or fixed embryos stained with DAPI and Phalloidin-Rhodamine. c and d, Liveborn pups derived from embryos stained with SPY650-DNA, n = 5 pups from 27 transferred blasts, (c) and SPY555-actin, n = 6 pups from 13 transferred blasts (d) following in utero transfer, shown are example litters. Scale bar, 10 µm.

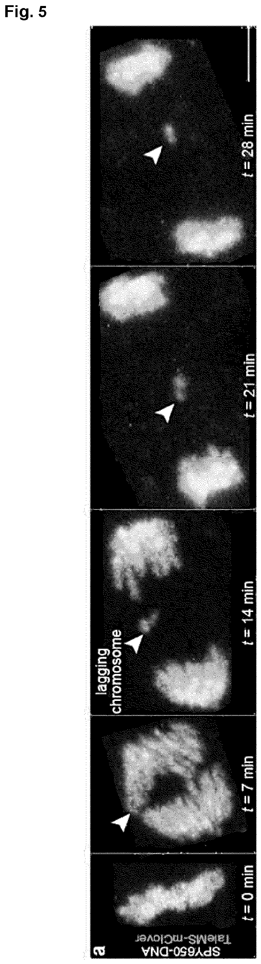

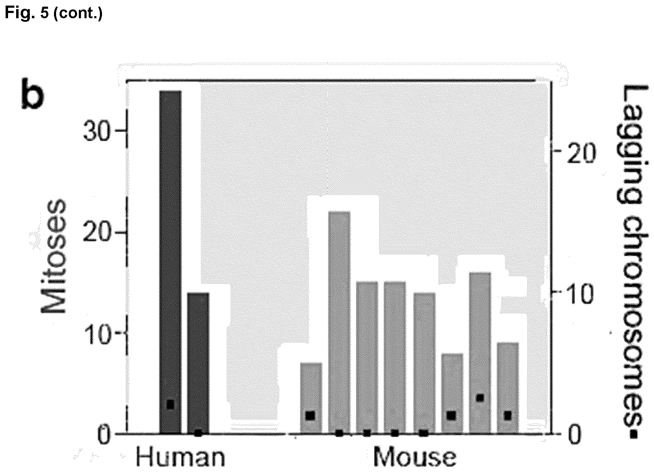

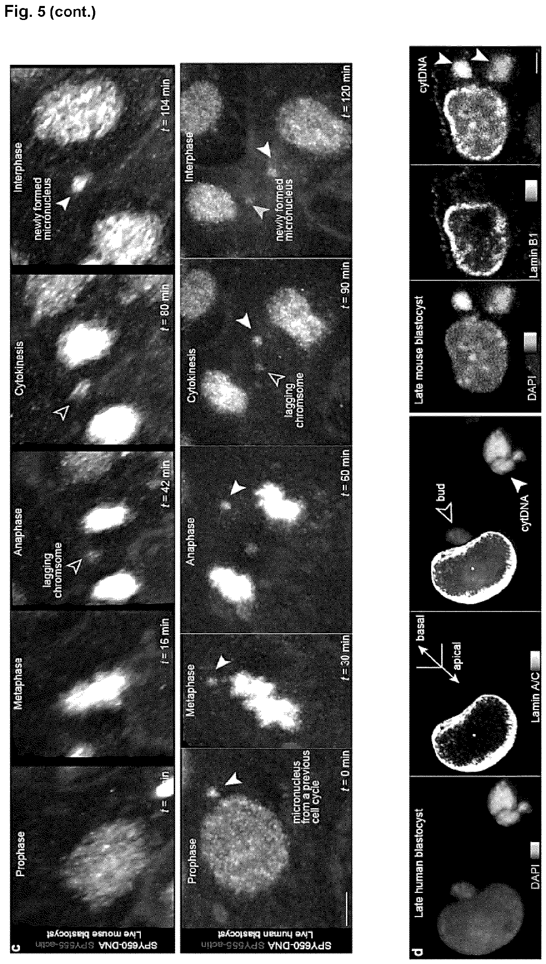

**Fig. 5****: Tracking chromosome segregation errors during mitosis in the living embryo. a,** Live-imaging in mouse 8-cell embryos microinjected with mRNA for the centromere marker TaleMS-mClover and stained with SPY650-DNA allow the identification of lagging chromosomes during mitosis. **b**, Quantification of lagging chromosomes relative to mitotic events in both mouse and human blastocysts. **c**, Live-imaging in mouse and human blastocysts with SPY650-DNA and SPY555-actin showing the formation of micronuclei in trophectoderm cells. **d**, Immunostaining for Lamin A/C in fixed human blastocysts and Lamin B1 in mouse blastocysts shows lack of Lamin staining around nuclear buds and cytDNA structures (labeled by DAPI). Scale bars, 10 µm.

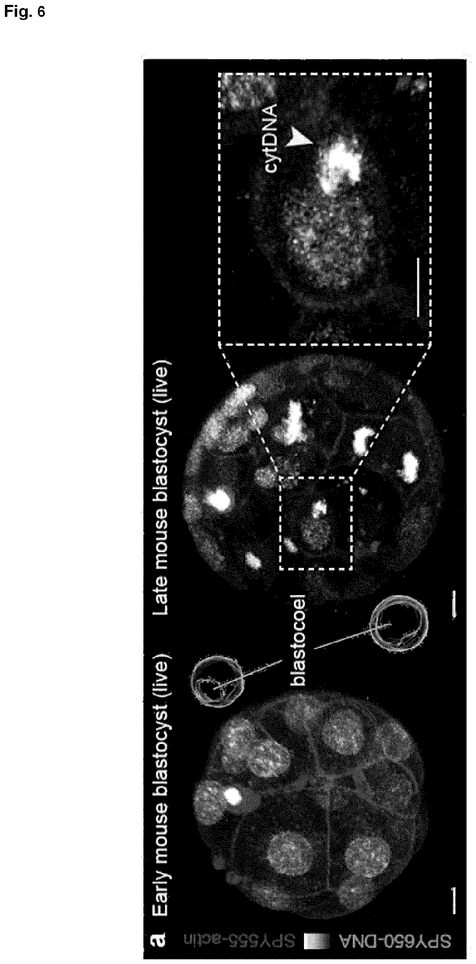

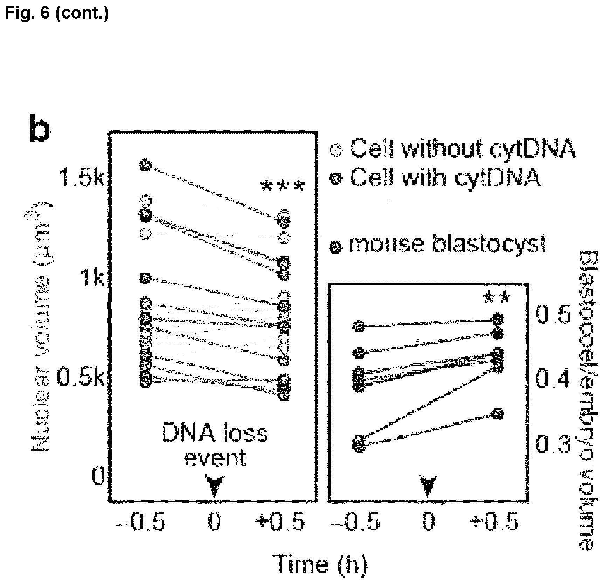

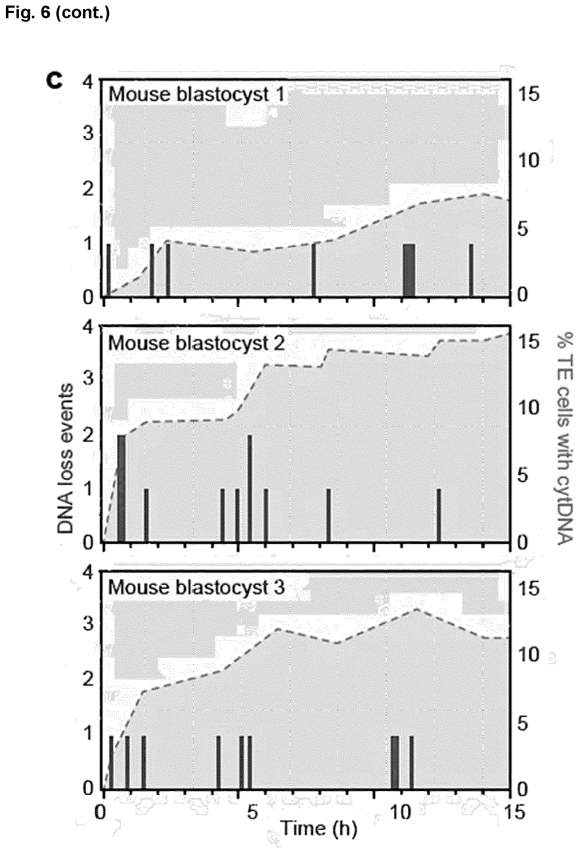

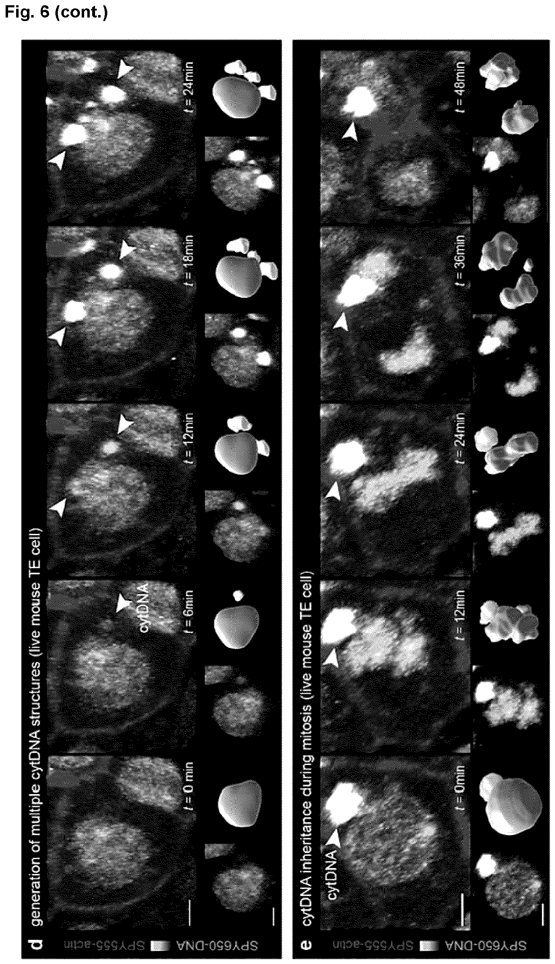

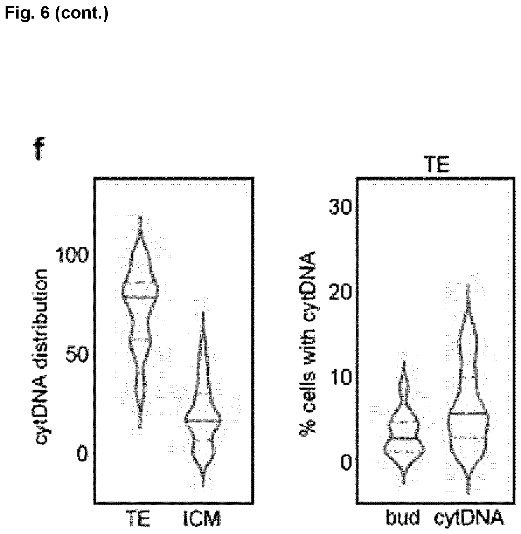

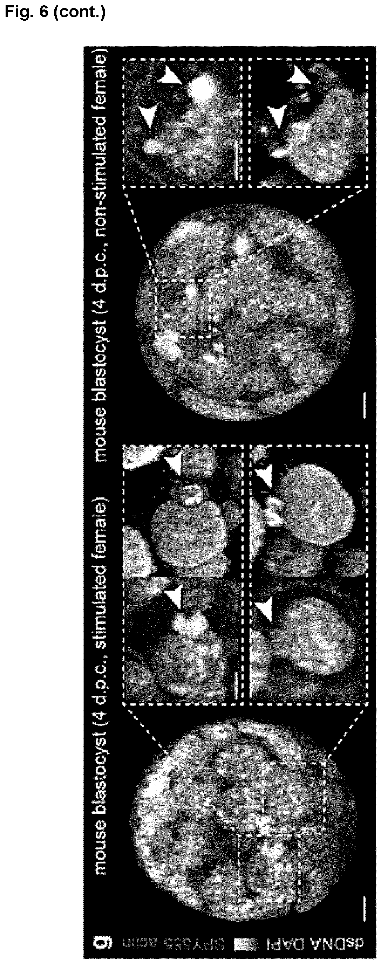

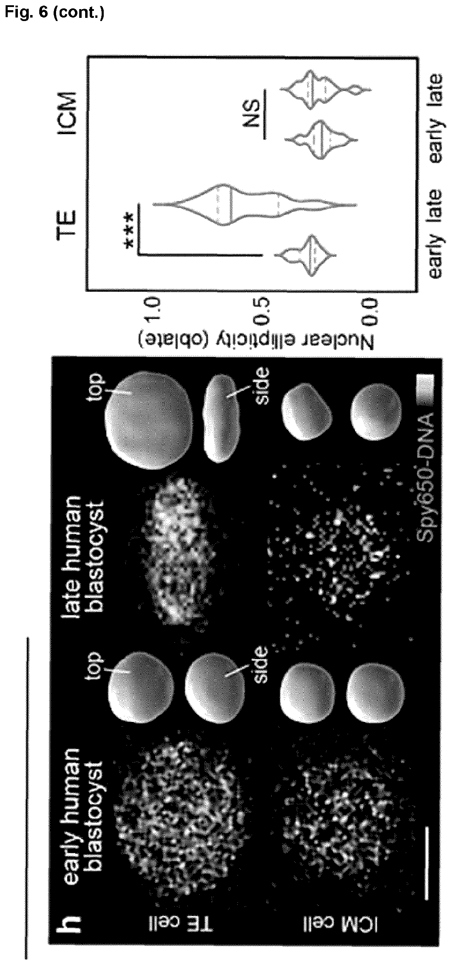

**Fig. 6****: Additional characterization of cytDNA structures. a**, Images of live mouse blastocysts reveal SPY650-DNA-labeled structures within the cytoplasm. Smaller insets show segmented embryos highlighting the increase in cavity volume at the advanced stage, **b**, Analysis of nuclear and blastocyst volumes pre-, and post-DNA loss (arrowhead indicates time of DNA loss). **c**, Quantification of nuclear loss events producing cytDNA in three different embryos over time. **d** and **e**, Live-imaging of mouse blastocysts with SPY650-DNA and SPY555-actin show the generation of multiple cytDNA structures and their inheritance during mitosis. **f**, Quantification of the distribution of cytDNA structures within the mouse blastocyst and the percentage of cells containing nuclear buds and cytDNA within the mouse trophectoderm. **g**, Detection of cytDNA structures in mouse blastocysts isolated from at 4 d.p.c. from the uterus of stimulated and non-stimulated pregnant females, fixed and stained with DAPI and with antibodies against dsDNA. **h**, Analysis of nuclear morphology in live embryos labeled with SPY650-DNA reveal flattening of trophectoderm cell nuclei in the advanced-stage human blastocyst. Quantification of nuclear ellipticity (oblate) serves as a proxy for flattening. TE, trophectoderm; ICM, inner cell mass. Scale bars, 10 µm.

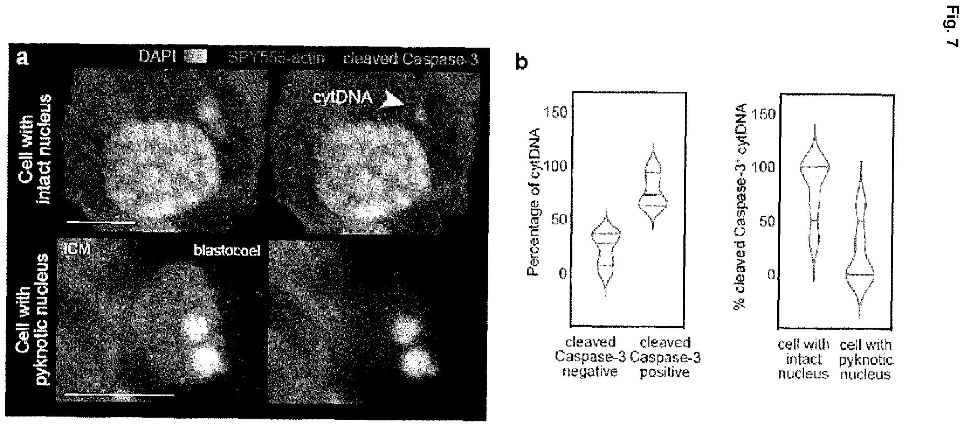

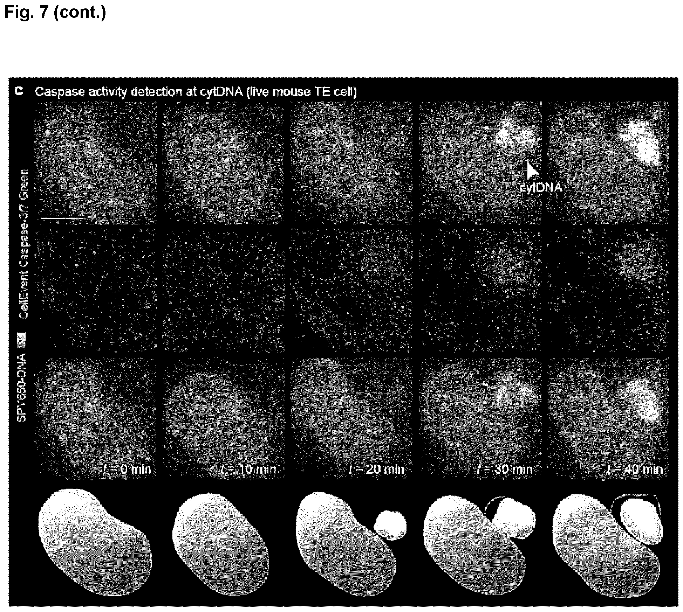

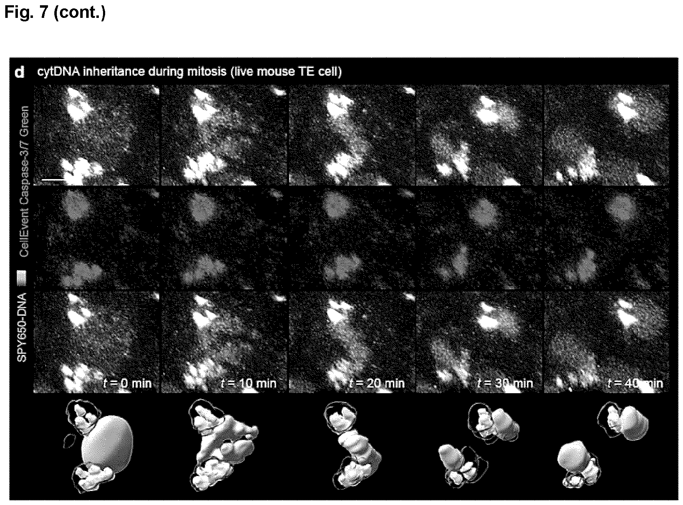

**Fig. 7****: cytDNA structures are surrounded by caspase activity but do not mark cells undergoing PCD. a**, Immunostaining for cleaved Caspase-3 with SPY555-actin and DAPI in fixed mouse blastocysts. Example of trophectoderm cell showing cleaved Caspase-3 surrounding a cytDNA structure. Note that the corresponding cell nucleus remains intact and does not display cleaved Caspase-3 staining (top panel). By contrast, the bottom panel shows an example of a pyknotic nucleus recognized by cleaved Caspase-3 undergoing PCD in the ICM. **b**, Quantification of cytDNA structures positive for cleaved Caspase-3 in cells displaying either an intact or pyknotic nucleus. **c**, Live-imaging of mouse blastocysts with SPY650-DNA, SPY555-actin and CellEvent Caspase-3/7 Green reveals caspase activity following the formation of cytDNA. **d**, Example of cell producing cytDNA detected by caspase activity and undergoing cell division. Note the inheritance of the cytDNA structures by the daughter cells. Scale bar, 10 µm.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value. The indicated value is also considered encompassed by the term about.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of does not exclude materials or steps that do not materially affect the basic and novel characteristics of the claim.

"Fluorescence" is the phenomenon in which light energy ("exciting light") is absorbed by a molecule resulting in the molecule becoming "excited". After a predescribed interval such as 1 minute-24 hours, the absorbed light energy is emitted by the excited molecule. The wavelength of the emitted light is typically at a longer wavelength than the exciting light. This emitted light is referred to as fluorescent light. A molecule that exhibits fluorescence is referred to as a "fluorophore." The relationship between wavelengths of light and degree of excitation of a given fluorophore at that wavelength is described by the "excitation spectrum" of the fluorophore. The excitation spectrum is also called the excitation wavelength range. The relationship between the wavelength of light and the intensity of the fluorescence emission at that wavelength is described by the emission spectrum or fluorescence spectrum of the fluorophore. The emission spectrum is also called the emitted wavelength range. The excitation maximum is the wavelength of exciting light at which fluorescence of the fluorophore reaches maximum intensity. The emission maximum is the wavelength of light emitted by the excited fluorophore when its fluorescence is at maximum intensity.

By "absorbance wavelength (A_{λ})" is referred herein as a measure of the capacity of a substance to absorb light of a specified wavelength. By "fluorescence wavelength (F_{λ})" is referred herein as the emission of light from a substance that has absorbed energy previously.

### DESCRIPTION OF THE EMBODIMENTS

Approaches that achieve non-invasive imaging of cells and embryos remain an open challenge. Enormous efforts have been devoted to find the appropriate method to study embryonic development and to identify the competent embryo, including methods that allow the identification of healthy embryos that do not carry genomic aberrations, such as aneuploidy. Pre-implantation genetic screening for aneuploidy seemed very promising; however, the invasiveness of the method itself, and the inherent problems in testing embryos at different stages of development may hamper the accuracy of the results. As a result, new, non-invasive and highly accurate methods that combine cells and embryo imaging monitoring with genetic and morphological assessment are desired.

The authors of the present invention describe herein a new method of time-lapse cell imaging, which combines two probes that stain nuclear DNA and actin. More specifically, incubation of cells with the dye SPY650-DNA, which labels genomic DNA, and SPY555-actin, which labels F-actin, produced high signal to noise ratios (see Fig. 4b), where the labelling pattern was equivalent to those produced using to invasive microinjection of mRNAs coding for fluorescently-tagged histone 2B (H2B-GFP) and utrophin-RFP (Utr-RFP) (Fig. 1a-d). The combination of fluorescent dyes with time-lapse imaging permitted the study of key events in the embryo dynamics underlying human preimplantation development, such as cell polarization, capturing main phases of mitosis, main changes in cell shape of the embryo, establishment of apical actin rings, tracking expansion and zippering of actin rings prior to blastocyst formation, etc., see Fig. 1 and 2. This method not only helps to monitor the key milestones during embryonic development, but also served expose important differences between human and mouse embryo development.

Furthermore, preimplantation embryos often contain a larger number of aneuploid cells than somatic tissues. Using the method described herein for embryo time-lapse imaging, it was found that events of nuclear DNA loss occur at the blastocyst level during embryo development, in both human and mouse (see Fig. 3). This is observed by the formation of bud-like structures labelled by SPY650-DNA protruding outwards from the nucleus, until they are lost into the cytoplasm, producing cytDNA (Fig. 3b). Strikingly, in some cases, the same cell nucleus underwent more than one DNA loss event producing cytDNA. Moreover, the majority of tracked cells containing cytDNA subsequently underwent mitosis (Fig. 6d). Therefore, thanks to the method of the invention, an additional process accounting for mosaic aneuploidy detected in trophectoderm biopsies was studied.

This new method is thus useful to bypass cell-labeling approaches relying on mRNA or DNA microinjection, and opens new paths for embryo development studies, that are highly valuable especially in assisted reproductive techniques.

In view of the above, **a first aspect** of the present invention relates to a method, from hereinafter referred to "the method of the present invention", for obtaining one or more fluorescent images of one or more cells, the method comprising the steps of incubating of the one or more cells with a first probe for fluorescence imaging of nuclear DNA and with a second probe for fluorescence imaging of another cellular component, preferably selected from cellular cortex, cell membrane, cortical actin filaments or actin, wherein the method comprises a step of visualizing the one or more cells stained with both probes by fluorescence microscopy. Preferably, the method of the present invention comprises the steps of:
(a) incubating the one or more cells with a first probe for fluorescence imaging of nuclear DNA, wherein the incubation produces a stain indicative of the presence of nuclear DNA,
(b) prior to, simultaneously to, or subsequently to step (a), incubating the one or more cells with a second probe for fluorescence imaging of another cellular component, preferably selected from cellular cortex, cell membrane, cortical actin filaments or actin, wherein the incubation produces a stain indicative of the presence of said component, and
(c) providing one or more images of the one or more cells that have been stained with both the first and the second probe, said one or more images being obtained by fluorescence microscopy.

In an embodiment, the method of the present invention is conducted during cultivation of the one or more cells, such as the one or more cells are positioned in a culture medium. Preferably, the method of the present invention comprises a step of culturing the one or more cells under conditions to promote survival, growth and/or development. Means for culturing cell populations are known in the art. The step of culturing the one or more cells may be performed before, during, or after the method of the present invention. Preferably this step is performed before incubating with the first and second probes. Preferably, the one or more cells are cultured under conditions to promote survival, growth and/or development throughout all the method.

In an embodiment, the first probe is characterized for comprising a molecule that is capable of binding to DNA, preferably nuclear DNA, preferably total nuclear DNA. The term "capable of binding" as used herein refers to the ability of a molecule to bind or target another molecule, such as DNA, and form a complex with it. Such binding may be by any chemical, physical or biological interaction between the molecules, including, but not limited, to any covalent, steric, agostic, electronic and ionic interaction between the molecules. Methods to determine whether a molecule is capable of binding to DNA are known in the art and include techniques such as DNA electrophoretic mobility shift assay (EMSA), aimed at observing how the complexes of molecule-DNA migrate more slowly than free DNA molecules when subjected to non-denaturing polyacrylamide or agarose gel electrophoresis. Other techniques suitable for study molecule-DNA complexes include DNA pull-down assays, DNA capture and detection assays, etc.

Preferably, the first probe is capable of specifically binding to DNA, preferably nuclear DNA. As used herein, the term "specifically binding" refers to the ability of a molecule to bind to a specific molecule, such as DNA, but not to wholly unrelated molecules. Thus, being capable of specifically binding to DNA means that the molecule comprised in the first probe only binds to DNA, preferably nuclear DNA, and not to other cellular compounds or structures. Preferably, the fluorophore used to specifically bind nuclear DNA does not bind to mitochondria DNA. In an embodiment, said molecule is selected from the group including, but not limited to, Hoechst stains, bisbenzimidie or a molecule comprising bisbenzimidie. Preferably, said molecule comprised in the first probe is capable of binding to the minor groove of the double-stranded DNA, preferably with a preference for sequences rich in adenine and thymine.

Preferably, the first probe comprises a Hoechst stain or dye comprising the formula 1 (C₂₅H₂₆N₆R): 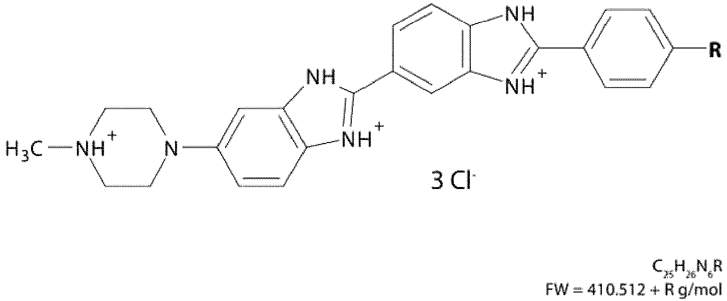 wherein R is selected from the group consisting of -OH (also named Hoechst 33258), -OCH2CH3 (also named Hoechst 33342), or -N(CH3)2 (also named Hoechst 34580).

Preferably, the first probe comprises a Hoechst stain, preferably selected from Hoechst 33258, Hoechst 33342, Hoechst 34580, Hoechst Janelia Fluor 646 (also named Hoechst JF 646, JF646-Hoechst or 2-(3-(Azetidin-1-ium-1-ylidene)-7-(azetidin-1-yl)-5,5-dimethyl-3,5-dihydrodibenzo[b,e]silin-10-yl)-4-((2-(2-(2-(4-(4-(5-(4-methylpiperazin-1-yl)-1H,3'H-[2,5'-bibenzo[d]imidazol]-2'-yl)phenoxy)butanamido)ethoxy)ethoxy)ethyl)carbamoyl)benzoate).

Preferably, the first probe comprises a bisbenzimide compound or a functional derivative of bisbenzimide. By "functional derivative" is referred herein a molecule has the same activity of the molecule to which is a functional derivative, irrespective or its structure. For instance, a functional derivative of bisbenzimide is a molecule has the same activity of bisbenzimidie, namely the capability of binding to nuclear DNA, although it may differ to bisbenzimidie in its molecular structure.

In an embodiment, the first probe also comprises a first fluorophore. Said first fluorophore may be the same molecule as the one with the capability of binding to DNA, or it may be a different molecule also comprised in the first probe. Preferably, said first fluorophore is excited at a wavelength that does not result toxic for a living cell, preferably a wavelength of more than 500 nm. Preferably, said first fluorophore has a maximum absorbance wavelength (A_{λ}) of between 700-600 nm and a maximum fluorescence wavelength (F_{λ}) of between 700-600 nm. More preferably, said first fluorophore has a maximum absorbance wavelength (A_{λ}) of 652 nm and a maximum fluorescence wavelength (F_{λ}) of 674 nm. Preferably, the fluorophore is selected from the group consisting of Cyanine 5, cyanine 5.5, APC, APC, APC and CY-7 conjugates, SYTO dyes, TOPO dyes, Miami Yellow, Pico Green, methylene blue, G-Dye 300, SPY650, Alexa Fluor 647, Janelia Fluor 646 or any functional derivatives thereof.

In another embodiment, the first fluorophore has a maximum absorbance wavelength (A_{λ}) of between 600-500 nm and a maximum fluorescence wavelength (F_{λ}) of between 600-500 nm. More preferably, said first fluorophore has a maximum absorbance wavelength (A_{λ}) of 555 nm and a maximum fluorescence wavelength (F_{λ}) of 580 nm.

Most preferably, the first probe is characterized for comprising a molecule that is capable of binding to DNA, preferably nuclear DNA, as defined in any of the above embodiments, and by comprising a first fluorophore as defined in any of the above embodiments, wherein the molecule that binds to DNA and the first fluorophore are different molecules and are conjugated to each other, preferably covalently. Even more preferably, the second probe comprises a bisbenzimide molecule or a functional derivative thereof or a molecule comprising bisbenzimidie, which is conjugated to a fluorophore that has a maximum absorbance wavelength (A_{λ}) of between 700-600 nm, preferably 652 nm, and a maximum fluorescence wavelength (F_{λ}) of between 700-600 nm, preferably 674 nm.

In an embodiment, the second probe is characterized for comprising a molecule that is capable of binding to actin, preferably F-actin. Methods to determine whether a molecule is capable of binding to actin are known in the art and include techniques such as co-sedimentation assays, pelleting assays, differential centrifugation assays, SDS-Page assays, etc, wherein the complexes resulting of the binding reaction (namely, the product resulting of actin bound to the molecule capable of binding to actin) are detected. Preferably, the second probe is capable of specifically bind to actin, preferably F-actine. Being capable of specifically bind to actin means that the molecule comprised in the second probe only binds to actine, preferably F-actine, and not to other cellular compounds. Preferably, the molecule capable of binding to actin, preferably F-actine, is selected from the group including, but not limited to, Jasplakinolide or phalloidin, or functional derivatives thereof. Preferably, the molecule that is capable of binding to actin promotes actin stabilization and/or polymerization.

Preferably, the second probe comprises a Jasplakinolide molecule, or a functional derivative of Jasplakinolide. Jasplakinolide is a macrocyclic peptide isolated from the marine sponge Jaspis johnstoni and is a potent inducer of actin polymerization in vitro by stimulating actin filament nucleation. By "functional derivative of Jasplakinolide" is referred herein a molecule has the same activity of Jasplakinolide, namely the capability of binding to actin, but it may differ to Jasplakinolide in its molecular structure. The molecular formula of Jasplakinolide is C₃₆H₄₅BrN₄O₆, corresponding to formula 2:

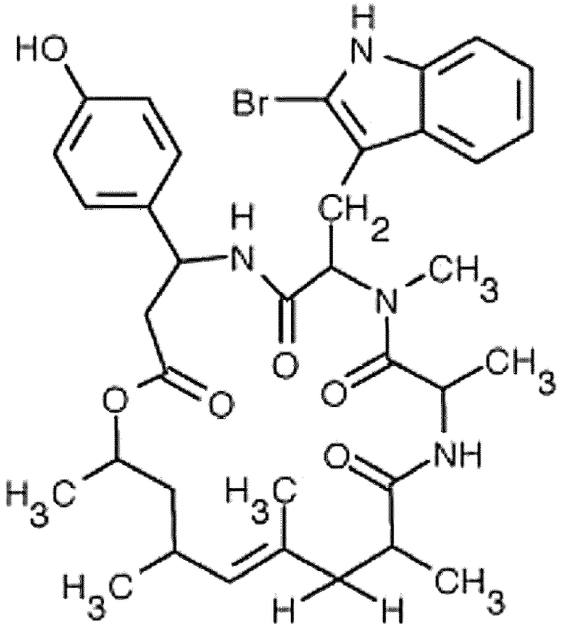

In an embodiment, the second probe comprises a second fluorophore. Said second fluorophore may be the same molecule as the one with the capability of binding to actin, or it may be a different molecule also comprised in the second probe. Preferably, said second fluorophore is excited at a wavelength that does not result toxic for a living cell, preferably a wavelength of more than 500 nm. Preferably, said second fluorophore has a maximum absorbance wavelength (A_{λ}) of between 600-500 nm and a maximum fluorescence wavelength (F_{λ}) of between 600-500 nm. More preferably, said second fluorophore has a maximum absorbance wavelength (A_{λ}) of 555 nm and a maximum fluorescence wavelength (F_{λ}) of 580 nm. In an embodiment, the fluorophore is selected from the list consisting of PE, Cy 3 and derivates, Texas Red, G-Dye 200, or functional derivatives thereof.

In another embodiment, the second fluorophore has a maximum absorbance wavelength (A_{λ}) of between 700-600 nm and a maximum fluorescence wavelength (F_{λ}) of between 700-600 nm. More preferably, said second fluorophore has a maximum absorbance wavelength (A_{λ}) of 652 nm and a maximum fluorescence wavelength (F_{λ}) of 674 nm.

Most preferably, the second probe is characterized for comprising a molecule that is capable of binding to actin, preferably F-actin, as defined above, and a second fluorophore as defined above, wherein the molecule that binds to actin and the second fluorophore are different molecules and are conjugated to each other, preferably covalently. Even more preferably, the second probe comprises Jasplakinolide or a functional derivative thereof conjugated to a fluorophore that has a maximum absorbance wavelength (A_{λ}) of between 600-500 nm, preferably 555 nm, and a maximum fluorescence wavelength (F_{λ}) of between 600-500 nm, preferably 580 nm.

It is noted that it is essential that the first and second fluorophores are different, i.e., they do not have overlapping absorbance wavelength. In a most preferred embodiment, one of the fluorophores, preferably the fluorophore of the first probe, has a maximum absorbance wavelength (A_{λ}) of 652 nm and a maximum fluorescence wavelength (F_{λ}) of 674 nm, and the other fluorophore, preferably the fluorophore of the second probe, has a maximum absorbance wavelength (A_{λ}) of 555 nm and a maximum fluorescence wavelength (F_{λ}) of 580 nm. In an embodiment, one of the fluorophore is selected from the list consisting of Cyanine 5, cyanine 5.5, APC, APC, APC and CY-7 conjugates, SYTO dyes, TOPO dyes, Miami Yellow, Pico Green, methylene blue, G-Dye 300, SPY650, Alexa Fluor 647, Janelia Fluor 646 or any functional derivatives thereof, and the other fluorophore is selected from the list consisting of PE, Cy 3 and derivates, Texas Red, G-Dye 200, SPY555, Alexa Fluor 555, Janelia Fluor 526, or functional derivatives thereof.

Thus, preferably, in the method of the present invention, the first probe comprises a molecule capable of binding to nuclear DNA conjugated to a first fluorophore, wherein the incubation produces a stain indicative of the presence of nuclear DNA; the second probe comprises a molecule capable of binding to actin conjugated to a second fluorophore; wherein the incubation produces a stain indicative of the presence of actin; wherein the first and the second fluorophores are different, and wherein one of the fluorophores, preferably the first fluorophore, has a maximum absorbance wavelength (A_{λ}) of 652 nm and a maximum fluorescence wavelength (F_{λ}) of 674 nm and the other fluorophore, preferably the second fluorophore, has a maximum absorbance wavelength (A_{λ}) of 555 nm and a maximum fluorescence wavelength (F_{λ}) of 580 nm

Preferably, the first and the second probes are cell permeable and can bind to DNA and actin, respectively, in live cells without negatively affecting their viability or without causing any damage to them. Thus, in a preferred embodiment, the staining probes are not toxic and allow for viability of cells after staining. By "cell permeable" is referred herein as the ability of the probe to pass into, out of, or through cells, or from one cell to another, i.e., the ability of the probe to pass in and out cell and nuclear membranes.

The incubation steps with the first and second probes may be performed sequentially or simultaneously. The first and/or the second probe may be provided in one or more staining compositions in any concentration useful to provide a desired level of contrast in the image of the one or more cells. Appropriate concentrations of a selected staining agent are readily determinable by those of skill in the art without resort to undue experimentation. For example, the probes may be diluted 1:100, 1:500, 1:1.000, 1:1.500, 1:2000, 1:3000, 1:4000, 1:5000, or any other concentration according to manufacturer's recommendations or culture conditions.

The period for which the one or more cells are incubated with the first and second probes is readily determined by those of skill in the art and is informed by the level of contrast desired in the images acquired from the stained one or more cells. It is generally preferred that the one or more cells remain in contact with the probes for a period of at least about 1 or 2 hour. Periods of at least about 1, 2, 3, 4, 5 hours, at least about 6 hours and at least about 12, 13, 14, 15, 16 or more hours are also of use in the methods of the invention.

Before, during, or after the incubation with the probes, the one or more cells (now doble-stained) may be optionally contacted with a buffer solution that may also be a wash solution. Thus, the method of the present invention may have one or more washing steps performed in before or after then other steps. An exemplary buffer is a buffered saline solution, e.g., phosphate buffered saline.

The result of the incubation step is that the one or more cells are double-stained. However, the method of the present invention is not limited to the use of only two probes, but the one or more cells may further be exposed to a third, four, etc staining probes.

The stained one or more cells are optionally transferred to one or a series of buffer solutions so as to remove extra staining and/or to create a density contrast between the cells and its bordering environment to facilitate distinguishing the nucleus and the cytoplasm. Then, the cells are prepared for fluorescence microscopy.

The images of the one or more cells are collected with a fluorescence microscope, preferably selected from WF, confocal microscopy, and TIRFM. Images that are acquired may be stored either on a continuous basis, as in live video, or on an intermittent basis, as in time lapse photography, where one or more cells are repeatedly imaged in a still picture. In an embodiment, the step of providing one or more images of the one or more cells by fluorescence microscopy is performed during a period of time by time-lapse image acquisition, in order to monitor closely the cells and to determine significant cellular events, such as morphological changes or nuclear DNA loss, as will be described below. Therefore, step (c) of the method may be performed for a period of time and may be a step of monitoring the one or more cells under suitable conditions for culturing them and for obtaining one or more images of them. The time interval between images can be varied depending on the amount of cell activity. For example, during active periods images could be taken as often as every few seconds or every minute, while during inactive periods images could be taken every 10 or 15 minutes or longer. Real-time image analysis on the captured images could be used to detect when and how to vary the time intervals.

In an embodiment, the one or more cells may also be visualized in real time or time-lapse to obtain the relevant information, for instance during at least 5, 10, 15, 20, 25, 30 mins, or at least 1 hour, at least 2 hours, at least 3 hours.

In an embodiment, the one or more cells may be cultured in standard culture dishes. Alternatively, they may be cultured in custom culture dishes, e.g., custom culture dishes with optical quality micro-wells. The hydrophobicity of the surface can be adjusted with plasma etching or another treatment to prevent bubbles from forming in the micro-wells when filled with media. The skilled person knows how to configure appropriate fluorescence microscopy and image acquisition setting suitable for the purposes of the method of the present invention.

The one or more images provided by the method of the present invention may be used for the three dimensional reconstruction of the one or more cells. The term "three dimensional reconstruction" has its general meaning in the art and relates to the reconstitution of the one or more cell in three dimensions.

Following image acquisition and visualization, the images are extracted and analysed for different cellular parameters, for example, cell size, morphology, thickness of the zona pellucida, degree of fragmentation, symmetry of daughter cells resulting from a cell division, time intervals between the first few mitoses, duration of cytokinesis, nuclear DNA and actin distribution, etc.

Preferably, the one or more cells are isolated cells. In some embodiments, the one or more cells are pluripotent cells. The term "pluripotent cell' is used herein to mean any cell that has the ability to differentiate into multiple types of cells in an organism. Preferably, the pluripotent cells are stem cells, oocytes, sperm cells, zygotes, embryos, or cancer cells. The term "stem cell' is used herein to refer to a cell or a population of cells which: (a) has the ability to self-renew, and (b) has the potential to give rise to diverse differentiated cell types. Frequently, a stem cell has the potential to give rise to multiple lineages of cells. As used herein, a stem cell may be a totipotent stem cell, e.g. a fertilized oocyte, which gives rise to all of the embryonic and extraembryonic tissues of an organism; a pluripotent stem cell, e.g. an embryonic stem (ES) cell, embryonic germ (EG) cell, or an induced pluripotent stem (iPS) cell, which gives rise to all of embryonic tissues of an organism, i.e. endoderm, mesoderm, and ectoderm lineages; a multipotent stem cell, e.g. a mesenchymal stem cell, which gives rise to at least two of the embryonic tissues of an organism, i.e. at least two of endoderm, mesoderm and ectoderm lineages, or it may be a tissue-specific stem cell, which gives rise to multiple types of differentiated cells of a particular tissue. Tissue-specific stem cells include tissue-specific embryonic cells, which give rise to the cells of a particular tissue, and somatic stem cells, which reside in adult tissues and can give rise to the cells of that tissue, e.g. neural stem cells, which give rise to all of the cells of the central nervous system, satellite cells, which give rise to skeletal muscle, and hematopoietic stem cells, which give rise to all of the cells of the hematopoietic system. The term "oocyte" is used herein to refer to an unfertilized female germ cell, or gamete The term "sperm cell" is used herein to refer to an unfertilized male germ cell, or gamete.

In an embodiment, the cells are a group of cells forming a tissue or an organ or parts thereof. In some embodiments, the cells are cancerous cells or cells derived from a tumour. In some embodiments, the cells are isolated cells from a biopsy, that may be a tumour biopsy or from a healthy donor, i.e., a healthy tissue or organ biopsy.

Most preferably, the one of more cells are comprised in or form an embryo. The term "embryo" used herein has its general meaning in the art and refers to the zygote that is formed when two haploid gametic cells, e.g., an oocyte and a sperm cell unite to form a diploid totipotent cell, e.g., a fertilized ovum, and to the embryo that results from the immediately subsequent cell divisions, i.e., embryonic cleavages, up through the morula, i.e. 16-cell stage and the blastocyst stage (with differentiated trophectoderm and inner cell mass). Accordingly, the term embryo is used herein to denote each of the stages fertilized oocyte, zygote, 2-cell, 4-cell, 8-cell, 16-cell, morula, blastocyst, expanded blastocyst and hatched blastocyst, as well as all stages in between (e.g., 3 -cell or 5- cell). In the context of the present invention, an embryo is preferably understood as an unborn or unhatched offspring in the process of development during the period from zygote, preferably from the fourth day after fertilization, to eight week after fertilization, after which it is termed a foetus. Thus, in an embodiment, an embryo comprises the earlies stages of development before a foetus. In the context of the present invention, an embryo is thus not a foetus.

Preferably, the embryo that is stained with the method of the present invention is in the blastocyst stage. As used herein, the term "blastocyst" refers to the structure formed in the early embryogenesis of mammals, after the formation of the morula, but before the formation of a foetus. It is characterized by comprising an inner cell mass (ICM), or embryoblast, which subsequently forms the foetus, and an outer layer of cells, or trophoblast, which later forms the placenta. The trophoblast is a layer that surrounds the inner cell mass and a fluid-filled blastocyst cavity known as the blastocoele. Thus, the blastocoel, trophoblast cells, and inner cell mass cells are hallmarks of the blastocyst. Preferably, the embryo is a human embryo which is in the blastocyst stage. Human embryos in the blastocyst stage are formed in a process called blastulation between 5-14 days after fertilization, and they are characterized by comprising between 24 up to 322 cells (blastula) with high capability of division, with a size of between 0.1-0.3 mm, preferably 0.2 mm, and comprising the trophectoderm (TE), the inner cell mass (ICM) and the blastocoel.

Embryos and pluripotent cells may be derived from any organism, e.g. any mammalian species, e.g. human, primate, equine, bovine, porcine, canine, rodent, feline, etc. They may be previously frozen, e.g. embryos cryopreserved at the 1 -cell stage and then thawed, or frozen and thawed oocytes and stem cells. Alternatively, they may be freshly prepared, e.g., embryos that are freshly prepared from oocytes by in vitro fertilization techniques; oocytes that are freshly harvested and/or freshly matured through in vitro maturation techniques or that are derived from pluripotent stem cells differentiated in vitro into germ cells and matured into oocytes; stem cells freshly prepared from the dissociation and culturing of tissues by methods known in the art; and the like. They may be cultured under any convenient conditions known in the art to promote survival, growth, and/or development of the sample to be assessed, e.g., for embryos, under conditions such as those used in the art of in vitro fertilization. The embryos/pluripotent cells are cultured in a suitable medium such as Continuous Single Culture Complete (CSC) media with human serum albumin (HAS), DMEM, Modified Dulbecco's Medium that has been supplemented with serum or serum substitute, amino acids, and growth factors tailored to the needs of the particular embryo/pluripotent cell being assessed with the method of the present invention. One or more developing embryos may be cultured in the same culture medium, e.g. between 1 and 30 embryos may be cultured per dish.

In some embodiments, the embryo is an isolated or an ex vivo embryo. Preferably, the method of the present invention, when applied to embryos, preferably human embryos, is performed on isolated embryos, preferably embryos that are collected after *in vitro* fertilization. In an embodiment, the embryos are live embryos and they do not suffer any damage throughout the method described herein.

In an embodiment, the method of the present invention is an *in vitro* method. In other embodiments, the method is an *in vivo* method. In other embodiments, the method is an *ex vivo* method. The term "*in vitro*" refers to a technique using samples (cells, tissues, organs, embryos) taken from the body but reproducing the same natural conditions. The term "*in vivo*" refers to a technique using samples (cells, tissues, organs, embryos) performed in or on a whole organism, such as a person. The term "*ex vivo*" refers to a technique using samples (cells, tissues, organs, embryos) taken from the body with minimal alteration of the natural conditions.

Preferably, especially in the case where the one or more cells are forming an embryo, the method of the present invention is an in vitro method that is non-destructive. By "Non-destructive method" is referred herein to a method that does not involves any damage or destruction of the one or more cells to which the method is applied. Thus, a non-destructive method does not negatively affect cell viability. By "cell viability" is referred herein as the measure of the proportion of live, healthy cells within a population. Thus, a method that is non-destructive does not alter the number of healthy cells. Methods to measure cell viability and proliferation are known in the art, such as live/dead assays, trypan blue staining, luminescent ATP assays etc.

The method of the present invention is also an in vitro method that is non-invasive. By "Non-invasive method" is referred herein as a method that does not require the alteration, incision, or break of the cell membrane. Thus, a non-invasive method leaves the cells intact or unbroken.

### Uses of the method of the present invention

The method provided herein may be used for treating one or more cells, preferably embryos, with the first and second probes defined herein, and optionally other probes, to provide images of one or more cells superior to those produced using currently recognized stains and/or staining techniques, and in a non-invasive way. As explained above, the method produces one or more images of the one or more cells providing a digital visualization with capabilities of taking a number of measurements, e.g., distance, area, and volume, as well as observing the nucleus, including DNA, and cytoplasm of the cell. This inherently digital method provides a practical tool for fields as diverse as reproductive biology, developmental biology, transgenic animal phenotyping, etc. Accordingly, the present invention has utility in the preparation of one or more cells of any type, for acquisition of images, and collection, and processing said images.

As shown in the Examples and discussed above, the method of the present invention is particularly useful to determine or identify the presence of nuclear DNA loss events in the one or more cells, which is possible due to the high imaging resolution that the combination of the first and second probes provide. By "nuclear DNA loss events" is referred herein as the process in which a part of the nuclear DNA is shed or expelled from the nucleus into the cytoplasm of a cell. DNA loss events are characterized by starting with the formation of a bud-like structure on the nuclear periphery of a cell, wherein said bud-like structure comprises nuclear DNA, then the bud-like structure eventually separates from the cell nucleus, carrying the part of the nuclear DNA with it and being released to the cytoplasm. Therefore, the nuclear DNA loss event is characterized by the presence of a structure, preferably a bud-like structure, comprising nuclear DNA protruding outwards from the nucleus into the cytoplasm of a cell, and results in a cell with lower amount of DNA in its nucleus. Preferably, said nuclear DNA loss events occur in the cells forming the trophectoderm layer during blastocyst formation. By "trophectoderm" is referred herein as the outer layer of the mammalian blastocyst after differentiation of the ectoderm, mesoderm, and endoderm. Preferably, the cells that present nuclear DNA loss events are localized in blastocysts, preferably in the trophectoderm layer of the blastocyst, and are characterized by having lower levels of keratin expression than a cell that is not undergoing DNA loss events. In an embodiment, the cells undergoing nuclear DNA loss events also present other markers such as the absence of keratin or lower expression of cytoskeletal components that protect the nucleus such as vimentin, microtubules or actine filaments, or any combination thereof.

In an embodiment, the method of the present invention is for determining whether one or more cells are losing or will lose part of their nuclear DNA, and it further comprises a step of visualizing on the one or more images provided by fluorescence microscopy the labelled nuclear DNA of the one or more cells and determining the presence or absence of nuclear DNA loss events in said cells, said events being characterized by the presence of labelled nuclear DNA protruding outwards from the nucleus into the cytoplasm of said one or more cells. Therefore, the method of the present invention can be used to determine the presence of nuclear DNA loss events in the one or more cells, the method comprising the steps of:
(a) incubating the one or more cells with a first probe for fluorescence imaging of nuclear DNA, wherein the incubation produces a stain indicative of the presence of nuclear DNA,
(b) prior to, simultaneously to, or subsequently to step (a), incubating the one or more cells with a second probe for fluorescence imaging of actin, wherein the incubation produces a stain indicative of the presence of actin,
(c) providing one or more images of the one or more cells that have been stained with both the first and the second probe, said one or more images being obtained by fluorescence microscopy, and
(d) simultaneously to, or subsequently to step (c), visualizing on the one or more images the nuclear morphology of the one or more cells and determining the presence or absence of nuclear DNA loss events, said the nuclear DNA loss events being characterized by the presence of nuclear DNA protruding outwards from the nucleus into the cytoplasm of the cell.

For the purposes of the method of the present invention, especially for the determination of the presence or absence of nuclear DNA loss events, a reference value or control cell may be used. The terms "reference" and "control" as used herein mean a standardized cell or cells used to interpret the presence or absence of nuclear DNA loss of a given test cell and assign a determination of whether said test cell presents or not nuclear DNA loss events. The reference or control may be a cell that is known to presents nuclear DNA loss events, and therefore may be a positive reference or control cell. Alternatively, the reference/control cell may be a cell known to not present nuclear DNA loss events, and therefore be a negative reference/control cell.

In some embodiments, the method for determining whether one or more cells are losing or will lose part of their nuclear DNA is performed on cells that are undergoing cell division, i.e., they are metabolic active and dividing. "Cell division" as used herein refers to both mitosis and meiosis processes. By "meiosis process" it is meant the cell cycle events that result in the production of gametes. In the first meiotic cell cycle, or meiosis I, a cell's chromosomes are duplicated and partitioned into two daughter cells. These daughter cells then divide in a second meiotic cell cycle, or meiosis II, that is not accompanied by DNA synthesis, resulting in gametes with a haploid number of chromosomes. Preferably, the cell is a mitotic cell. By a "mitotic cell", it is meant a cells where events that result in the duplication of a cell's chromosomes and the division of those chromosomes and a cell's cytoplasmic matter into two daughter cells are happening. The mitotic cell cycle is a cell undergoing mitosis. In interphase, the cell grows and replicates its DNA. In mitosis, the cell initiates and completes cell division, first partitioning its nuclear material, and then dividing its cytoplasmic material and its partitioned nuclear material (cytokinesis) into two separate cells. Preferably, the method for determining whether one or more cells are losing or will lose part of their nuclear DNA is performed on cells that are comprised in an embryo, preferably a mammalian embryo, most preferably a human embryo.

Therefore, in a preferred embodiment, the method is performed on mammalian embryo, wherein said mammalian embryo is cultured under conditions to promote survival, growth and/or development, and wherein the method comprises a step (d) of visualizing on the one or more images provided by fluorescence microscopy the labelled nuclear DNA of the one or more cells and determining the presence or absence of nuclear DNA loss events in said cells, said events being characterized by the presence of labelled nuclear DNA protruding outwards from the nucleus into the cytoplasm of said one or more cells.

The method of the present invention may also be used to determine the genetic quality of the one or more cells, as it assess whether the nucleus of said cells has lost DNA. In the present context, the genetic quality is preferably a quality relating to whether the one or more cells are in a situation of aneuploidy or not, i.e., whether they have undergone nuclear DNA loss. By "nuclear DNA loss" is referred herein to the absence or loss of part of the genomic DNA from the nucleus, and it includes loss of one or more full chromosomes as well as loss of parts of chromosomes or chromosomal segments (as whole chromosomes could maybe break off with some parts remaining in the nucleus and other parts shed to the cytoplasm). Thus, in the context of the present invention, nuclear DNA loss is not only referred to losses of entire chromosomes, but also losses of parts or segments of a chromosome. A cell that has undergone a process of nuclear DNA loss is a cell having aneuploidy. Hence, an aneuploid cell is a cell that does not have the correct amount of genetic material for its species, and therefore has poor genetic quality. Therefore, aneuploid cells are the opposite of trueploid or euploid cell. For humans, for instance, an aneuploid cell is a cell that has not a set of 46 complete chromosomes, but has less or more than 46 chromosomes, such as 45 or 47, or has lost part of some of its chromosomes. Accordingly, the method of the present invention may also be used as a method for diagnosing aneuploidy in one or more cells, the method comprising the steps of:
(a) incubating the one or more cells with a first probe for fluorescence imaging of nuclear DNA, wherein the incubation produces a stain indicative of the presence of nuclear DNA,
(b) prior to, simultaneously to, or subsequently to step (a), incubating the one or more cells with a second probe for fluorescence imaging of actin, wherein the incubation produces a stain indicative of the presence of actin,
(c) providing one or more images of the one or more cells that have been stained with both the first and the second probe, said one or more images being obtained by fluorescence microscopy, and
(d) simultaneously to, or subsequently to step (c), visualizing on the one or more images the nuclear morphology of the one or more cells and determining the presence or absence of nuclear DNA loss events, the presence of nuclear DNA loss events being characterized by the presence of nuclear DNA protruding outwards from the nucleus into the cytoplasm of the cell,
wherein the presence of nuclear DNA loss events in the one or more cells is indicative of the one or more cells having aneuploidy.

In an embodiment, the one or more cells are comprised in an embryo, preferably a mammalian embryo, most preferably a human embryo, and thus the method disclosed herein may be used as a method for diagnosing aneuploidy in an embryo, wherein the embryo is diagnosed with aneuploidy if nuclear DNA loss events are determined in at least one of its cells comprised in the embryo. Hence, in some embodiments, the method of the invention is for determining and/or diagnosing whether an embryo presents aneuploidy, wherein the embryo is cultured under conditions to promote survival, growth and/or development, and wherein the method comprises the steps of:
(a) incubating the embryo with a first probe for fluorescence imaging of nuclear DNA, wherein the incubation produces a stain indicative of the presence of nuclear DNA,
(b) prior to, simultaneously to, or subsequently to step (a), incubating the embryo with a second probe for fluorescence imaging of actin, wherein the incubation produces a stain indicative of the presence of actin, and
(c) providing one or more images of the cells comprised in the embryo that have been stained with both the first and the second probe, said one or more images being obtained by fluorescence microscopy,
(d) simultaneously to, or subsequently to step (c), visualizing on the one or more images the nuclear morphology of the individual cells comprised in the embryo and determining the presence or absence of nuclear DNA loss events in said cells, said nuclear DNA loss events being characterized by the presence of labelled nuclear DNA protruding outwards from the nucleus into the cytoplasm of the cell, and
wherein the visualization of nuclear DNA loss events in at least one of the cells comprised in the embryo is indicative that said embryo presents or will present aneuploidy. In an embodiment, if all the cells of an embryo are aneuploid (i.e., nuclear DNA loss events are observed in all of them), then embryonic aneuploidy is diagnosed. In another embodiment, if some of the cells of an embryo are aneuploid, and some of them are euploid, then the embryo is diagnosed with embryonic mosaic aneuploidy. Preferably, the embryo is a live isolated human embryo.

In an embodiment, the diagnosis of aneuploidy in an embryo or in one or more cells is indicative of poor genetic quality. Determining the genetic quality of an embryo allows the assessment of the ability of an embryo to perform successfully either or both in terms of conferring a high pregnancy rate and/or resulting in a healthy individual. In an embodiment, the presence of aneuploidy in an embryo may be used as a specific exclusion criterion for transplantation, as it points out to low genetic quality. Preferably, the diagnosis of aneuploidy in an embryo is performed when the embryo is at a blastocyst stage, preferably during the morphological events in the embryo development that take place when the trophectoderm layer becomes constrained during cavity expansion in the blastocyst. With this purpose, it is preferred that the images are taken during two or more time intervals thereby providing time-lapse images of the mammalian embryo. Hence, it is preferred that the method of the present invention, preferably steps (c) and (d) (i.e., steps of providing one or more images and step of visualizing the labelled nuclear DNA on them) of the method of the present invention, is performed for two or more time intervals thereby providing time-lapse images of the mammalian embryo, and wherein said time intervals are performed during the morphological events in the embryo development that take place when embryo is in blastocyst stage, preferably when the trophectoderm layer becomes constrained during cavity expansion in the blastocyst.

Following diagnosis of aneuploidy, if the sample is a group of cells, such as an embryo, the method of the present invention can be used for providing a prognosis for aneuploidy, wherein the prognosis comprises determining whether the aneuploidy is severe or not depending on the number of cells that have been identified with nuclear DNA loss events according to the present invention. The term "prognosis for aneuploidy " refers to the procedure by which a prediction of events that occur in the development or course of aneuploidy is established, including an increase in the severity of the aneuploidy. Thus, in an embodiment, the method of the present invention is a method for prognosing the severity of aneuploidy in a group of cells, the method comprising performing the method of the present invention on said cells and measuring how many of them have or are having nuclear DNA loss events, wherein a severe aneuploidy is determined if the majority of the cells visualized according to the method of the present invention have experienced or are experiencing nuclear DNA loss events. The method of the invention offers a fast way to predict the viability of embryos at an early stage and could therefore improve the prospects for in vitro fertilization techniques.

The method of the present invention can also be useful for evaluating the risk of developing aneuploidy in one or more cells, preferably in an embryo. By "evaluating the risk of developing aneuploidy" is referred herein as the likelihood of having aneuploidy. In an embodiment, the method of the present invention is a method for evaluating the risk of developing aneuploidy in one or more cells, the method comprising performing the steps of the method of the present invention on said one or more cells and identifying incipient or already formed bud-like structures comprising stained nuclear DNA protruding or budding from the cell nucleus, being the presence of said structures indicative of a high risk of developing aneuploidy.

As explained above and shown in the Examples (see, e.g., Figs 1 and 2), the invention also provides a practical tool for imaging and monitoring embryo development, including observing the cell dynamics underlying cleavage stage, where the changing positions of the cell boundaries (i.e., cell membranes) as a consequence of cellular movement causes a range parameters derived from the different images. Hence, the method of the present invention may also be used for visualizing three-dimensional (3D) embryos, visualization of central events occurring during preimplantation development, including: capturing the main phases of mitosis (Fig. 1b); visualizing the major changes in cell shape that characterize embryo compaction at the 8-cell stage (Fig. 1c); detecting cell polarization at the 8-cell stage identified by the formation of the apical domain, establishment of apical F-actin rings at the 16-cell stage (Fig. 1d), tracking the expansion and zippering of F-actin rings, which seal the embryo prior to blastocyst formation (Fig. 1d), detecting the first internalized cells within the 16-cell embryo, which will form the ICM (Fig. 1e), and visualizing blastocyst expansion and hatching from the zona pellucida (Fig. 1f).

Hence, in some embodiments, the method of the invention is helpful for visualizing and monitoring embryo development, and/or for detecting developmental abnormalities, wherein said mammalian embryo is cultured under conditions to promote survival, growth and/or development, and wherein the one or more images provided in step (c) of the method of the present invention are analysed to determine whether the embryo presents developmental abnormalities as compared to one or more control embryos. As used herein, developmental abnormalities or defects include defects in, e.g., mitosis, embryo compaction, enrichment of F-actin at the apical region or cortex of the cells during the time of embryo polarization, formation of the inner mass and trophectoderm, embryo cavitation and blastocyst expansion, blastocyst hatching from the zona pelucida process, or any combination thereof.

A reference or control embryo maybe a positive control embryo which is known to be a healthy embryo. Alternatively, the reference or control embryo may be a negative control embryo in which case it is an embryo that is known to present developmental abnormalities.

Preferably, the method for visualizing and monitoring embryo development, or for detecting developmental abnormalities comprises the steps of:
(a) incubating the embryo with a first probe for fluorescence imaging of nuclear DNA, wherein the incubation produces a stain indicative of the presence of nuclear DNA,
(b) prior to, simultaneously to, or subsequently to step (a), incubating the embryo with a second probe for fluorescence imaging of actin, wherein the incubation produces a stain indicative of the presence of actin,
(c) providing one or more images of the cells comprised in the embryo that have been stained with both the first and the second probe, said one or more images being obtained by fluorescence microscopy, and
(d) simultaneously to, or subsequently to step (c), visualizing on the one or more images the nuclear and cell morphology of the cells comprised in the embryo, and optionally determining the presence or absence of developmental abnormalities or defects in said embryo as compared with a reference or control embryo,
wherein the embryo is cultured under conditions to promote survival, growth and/or development.

Since the method allows the visualization of embryo development and the detection of developmental abnormalities or defects, the method can also be used for predicting the developmental potential of one or more embryos. The "developmental potential" of an embryo in the context of the present invention is a prediction of the ability of the embryo to subsequently develop successfully into a healthy individual, for instance in an individual without aneuploidy or morphological abnormalities. In some embodiments the prediction of developmental potential is based on the determination of the presence or absence of embryonic aneuploidy following the method of the present invention in conjunction with other parameters that are known to be predictive of developmental success, such as the absence of developmental abnormalities as compared to a control embryo. For example, taking measurements of the cytoplasmic movements and cell morphology and/or periodic changes in the shape of the embryo over time, which is also possible with the imaging method of the present invention. Assessments made by the method of the invention may also find use in ranking embryos for their developmental potential. For example, in some instances, multiple embryos may be capable of developing into healthy blastocysts, i.e. will have good developmental potential. However, some embryos will be more likely to achieve the blastocysts stage or a higher genetic quality blastocyst than other, i.e. they will have better developmental potential than other embryos. In such methods, the presence or absence of nuclear DNA loss events or aneuploidy or any developmental abnormality for each embryonic cell is measured to determine the developmental potential of the embryos relative to one another. Said determination may be employed to compare embryos to one another to determine their developmental potential. In this way, a practitioner assessing, for example, multiple zygotes/embryos, can choose only the best quality embryos, i.e. those with the best developmental potential, to transfer so as to maximize the chance of success of a full term pregnancy while minimizing risk. Information on the developmental potential of oocytes obtained by the methods of the invention can guide the practitioner's selection of ooctyes to fertilize, resulting in higher probability of success in deriving blastocysts from these oocytes.

Assessments made by following the methods of the invention may also find use in determining the developmental potential of other cells, such as oocytes that are matured and cultured in vitro. Likewise, information on the developmental potential of other cells, such as stem cells, can inform the practitioner's selection of stem cells to use in procedures to, e.g., reconstitute or replace a tissue in vivo in a subject in need thereof.

The method of the present invention may be used to guide clinical decisions, e.g., whether or not to transfer an embryo, or whether or not to transplant a culture cell or cells. As used herein the term "clinical decision" refers to any decision to take or not take an action that has an outcome that affects the health or survival of the embryo or the cells. For example, in order to increase pregnancy rates, clinicians often transfer multiple embryos into patients, potentially resulting in multiple pregnancies that pose health risks to both the mother and foetuses. Using results obtained from the methods of the invention, the genetic and morphological quality of embryos being transferred to develop into foetuses is determined prior to transplantation, allowing the practitioner to decide how many embryos to transfer so as to maximize the chance of success of a full term pregnancy while minimizing risk. In an embodiment, a clinical decision refers to a decision to implant or not the embryo of in the uterus of the patient. In particular the method of the present invention will help embryologist to avoid the transfer in uterus of embryos with a poor potential for pregnancy outcome.

The method of the present invention may also be used in a method, preferably an in vitro method, of selecting one or more embryos from a plurality of embryos, the method comprising monitoring the presence or absence of nuclear DNA loss events and/or the presence or absence or developmental abnormalities during development in an embryo by carrying out the method of the present invention and selecting one or more embryos based on their absence of nuclear DNA loss events and/or the absence of developmental abnormalities. Other factors may also be taken into account when selecting the embryo(s). Some of them include (1) shape of the embryo including number of blastomers and degree of fragmentation; (2) presence and quality of a zona pellucida; (3) size; (4) colour and texture; (5) knowledge of the age of the embryo in relation to its developmental stage, and (6) blastomere membrane integrity.

Also provided is a method, preferably an in vitro method, of assisted reproduction, the method comprising determining the presence or absence of nuclear DNA loss events and/or the presence or absence or developmental abnormalities in one or more embryos by carrying out the method of the present invention, and selecting one or more embryo(s) based on the absence of nuclear DNA loss events and/or developmental abnormalities and subsequently transferring said selected embryo(s) to a maternal recipient. "Transfer to a maternal recipient" means the transfer of a healthy embryo into a would-be mother with the aim of a resulting pregnancy.

The method of the present invention may also be used in a method of in vitro fertilization, comprising fertilizing an egg, preferably mammalian egg, and monitoring the presence or absence of nuclear DNA loss events and/or the presence or absence or developmental abnormalities in one or more embryos using the method described herein. In vitro fertilized eggs or embryos that do not present any nuclear DNA loss events and/or developmental abnormalities in any of its cells may be selected for transfer to a maternal recipient, thereby improving successful pregnancy rates.

The method of the present invention may also be used in a method, preferably an in vitro method, for enhancing the pregnancy outcome of a patient comprising the steps consisting of i) providing a plurality of embryos, ii) determining the quality of the embryo by performing the method according to the invention, particularly determining whether or not the embryo suffers from aneuploidy and/or developmental abnormalities, iii) selecting the most competent embryo, and iv) implanting the embryo selected at step iii) in the uterus of said patient. By most competent embryo is referred herein as the embryo with the highest quality, i.e., with no aneuploidy nor developmental abnormalities.

The embryos that are assessed according to the method of the invention may be intended for use in subsequent downstream procedures, such transfer to a maternal recipient resulting in pregnancy. It is therefore important to minimise any damage to the embryos whilst carrying out the method. The method of the invention when applied to embryos is thus non-invasive and non-destructive, namely it does not damage or harm the embryos. Therefore, the method does not affect the viability or development of the embryo. The medium where the embryos are cultured is maintained under optimal conditions for the embryos to develop. The medium may be buffered, for example with HEPES, to keep a constant pH. An environmental chamber can be used to maintain a steady pH. The environmental chamber may provide CO₂, for example 5% CO₂ to keep the pH of the medium at the correct level. An environmental chamber may also be used to regulate the temperature of the embryos during the method of the present invention. For example, the temperature may be maintained at 35 to 40 °C, or 37 to 38 °C or at. a out 37.5 °C. The temperature may also be controlled by using a heated stage.

Further, the images obtained in step (c) by fluorescence microscopy are provided using light that does not damage the embryos. Appropriate, low phototoxicity wavelengths are selected by the use of the fluorophores defined above, which do not damage the embryo. The first and second probe, as defined above, are non-cytotoxic, nor harmful for the embryos.

The above uses of the imaging method of the present invention may include evaluating image series of developing embryos (time-lapse images). The resulting one or more images can be used to quantify the amount of change occurring in the embryo between consecutive frames in an image series. Therefore, in an embodiment, the step of providing one or more images is performed for two or more time intervals on an embryo. Preferably, said time intervals are performed in an embryo during the morphological events in the embryo development that take place when the trophectoderm cells become mechanically constrained during cavity expansion. Preferably, method of the present invention is applied to an embryo at the 8-cell, 9-cell, 10-cell, 11-cell, 12-cell, 13-cell, 14-cell, 15-cell, or 16-cell stage. Preferably, the method of the present invention is applied to an embryo, preferably a human embryo, during the late morula stage to the blastocyst stage, i.e., before it becomes a foetus.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

### Combination of the first and second probe

The present invention also provides the combined use of the first and second probe as defined in the first aspect of the present invention or any of its embodiments for carrying out the method of the present invention or any of its uses as defined above. Preferably, the present invention provides the in vitro use of the first probe in combination with the second probe as defined in the above aspect, for one or more of the following applications:
- obtaining a fluorescent image of one or more cells, preferably an embryo,
- determining the presence or absence of nuclear DNA loss events in one or more cells, preferably an embryo,
- determining the genetic quality and/or the presence of aneuploidy in one or more cells,
- determining and/or diagnosing whether an embryo presents aneuploidy,
- providing a prognosis for aneuploidy in a group of cells, preferably an embryo,
- evaluating the risk of developing aneuploidy in one or more cells, preferably in an embryo,
- predicting the developmental potential of one or more cells, preferably an embryo,
- selecting one or more embryos from a plurality of embryos for downstream procedures, such transfer to a maternal recipient resulting in pregnancy,
- in a method of assisted reproduction, and
- in a method of in vitro fertilization,
- or any combination thereof.

### Systems

In a further aspect, the invention provides a system for carrying out the method of the invention, the system comprising one or more sensors for capturing images of the one or more cells or the embryo, and at least one processor in communication with the at least one sensor, the processor being programmed with computer-readable instructions to transform said images into a determination of the presence or absence of nuclear DNA loss events in the one or more cells or the embryo or the determination of developmental abnormalities in the embryo.

The invention further relates to a data carrier comprising a computer program directly loadable in the memory of a digital processing device and comprising computer code portions constituting means for executing the method of the invention as described above. The data carrier may be a magnetic or optical disk or in the shape of an electronic card. Further provided is a computer program product comprising instructions such that when processed by a processor, configures it to perform the method defined in the first aspect.

In addition, in some cases, a computer may be used to acquire microscope images of the one or more cells, preferably of the embryo, and/or to determine whether said cells or embryo have suffered from nuclear DNA loss events or if the embryo presents developmental abnormalities. For example, images obtained by the method of the present invention may be analysed by a computer or artificial intelligence software to determine the presence or absence of nuclear DNA loss events in one or more cells. The computer may be any suitable computer, e.g., a general-purpose computer or a specially-built computer, etc. Therefore, in a further aspect, the present invention also provides to a computer implemented method for carrying out the method defined in the first aspect of the present invention.

Uses of the system for carrying out the method defined under the first aspect are also comprised herein.

### Kits and uses of kits

In another aspect, the present invention provides a kit comprising a first and a second probe, as defined above in the first aspect of the present invention or any of its embodiments. This aspect also includes uses of the kit for carrying out the method defined under the first aspect.

In addition to the above components, the kits may further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

### Example 1

### Methods

### Mouse embryo work

Superovulated wild-type female mice (Hsd:NSA(CF-1)) were used to obtain embryos. Embryos were flushed from oviducts with M2 medium (Merck) and cultured in KSOM+AA (Merck) at 37°C and 5% CO₂ covered by mineral oil (Sigma). Five-week-old female mice were superovulated using 5 IU of pregnant mare serum (PMS, National Hormone and Peptide Program) followed by 5 IU of recombinant chorionic gonadotrophin (CG, National Hormone and Peptide Program) given intraperitoneally 48 h after and immediately before mating. Two-cell stage embryos were flushed from oviducts with M2 medium (Merck) and cultured in KSOM+AA (Merck) covered by mineral oil (Sigma), at 37 °C and 5% CO₂. For some experiments, blastocysts were isolated from the uterus at 4 d.p.c. from either non-stimulated or stimulated females and fixed.

### Human embryo work

Previously vitrified embryos were thawed according to the manufacturer's protocol (90137-SO-Vit Kit-Thaw, FUJIFILM Irvine Scientific, USA) and cultured for 1 h in individual drops of 75 µl of Continuous Single Culture Complete (CSC) media with human serum albumin (HSA) (FUJIFILM Irvine Scientific, USA), covered with mineral oil in an incubator at 37°C, 7% CO₂ and 6% O₂ until further staining.

### Non-Surgical Embryo Transfer (NSET)

Mouse blastocysts were transferred to CF1 pseudopregnant females via NSET using the NSET Non-Surgical Embryo Transfer Device (ParaTechs Corporation) according to the manufacturer's guidelines. Each recipient female received 12-20 blastocysts. The embryos were either untreated or stained with SPY650-DNA or SPY555-DNA prior to transfer. The mice were then allowed to deliver full-term pups.

### DNA and mRNA

DNA constructs were cloned into pCS2+ vector for mRNA production. The mMESSAGE mMACHINE^{®} SP6 kit (Ambion) was used to synthesize RNA using linearized plasmids as templates then purified using the RNAeasy kit (Qiagen) following manufacturer's instructions. Embryos were microinjected with 0.1 to 0.3 pL RNA diluted in injection buffer (5 mM Tris, 5 mM NaCl, 0.1mM EDTA) with a FemtoJet (Eppendorf) as follows: 10 ng µl⁻1 H2B-RFP; 5 ng µl⁻¹ H2B-GFP; 75 ng µl⁻¹ Utrophin-RFP; 100 ng µl⁻¹ TaleMS-mClover.

### siRNAs (Qiagen)

Mm_Krt2-8_1 (AACCATGTACCAGATTAAGTA, 200 nM)
Mm_Krt2-8_2 (ATGGATGGCATCATCGCTGAA, 200 nM)
Mm_Krt1-18_1 (CAGAGTGGTGTCCGAGACTAA, 200 nM)
Mm_Krt1-18_3 (CCGGGAACATCTGGAGAAGAA, 200 nM)
AIIStars negative control siRNA (Sequence undisclosed by Qiagen, 800 nM)

### Live embryo imaging

Live mouse and human embryos were stained with the live cell stain probes SPY555-Actin (1:2,000, Spirochrome), SPY650-DNA (1:1,000, Spirochrome), SPY555-DNA (1:1,000, Spirochrome), SPY650-FastAct (1:2000, Spirochrome), Cell Event Caspase-3/7 Green Detection Reagent (1:1,000, Invitrogen) for 1-2 h depending on the embryo stage, then imaged in a 1:5,000-10,000 solution in KSOM. For imaging, embryos were cultured in LabTek chambers (Nunc) at 37C and 5% CO₂ in an incubator adapted for the microscope system (Leica SP8 or Nikon A1RHD25). Mouse embryos were imaged using a laser scanning confocal (Leica SP8) with water Apochromat 40X 1.1 NA objectives and highly sensitive HyD detectors (Leica). Human embryos were imaged on a Nikon A1RHD25 point scanning confocal microscope in the Nikon Biolmaging Lab (NBIL, Cambridge MA).

### Staining and immunofluorescence

For immunostaining, embryos were fixed with 4% paraformaldehyde in DPBS-0.1 % Triton X-100 for 30 min at room temperature, permeabilized in DPBS-0.5% Triton X-100 for 30 min, incubated in blocking solution (10% fetal bovine serum in DPBS-0.2% Triton X-100) for 1 h and incubated with primary antibodies in blocking solution overnight at 4°C: dsDNA (1:1,000, Abcam), Keratin-8 (1:100, DSHB) , Lamin-A/C (1:200, Santa Cruz), Lamin-B1 (1:200, Protein Tech), cleaved Caspase-3 (1:200, Cell Signaling). Embryos were rinsed in DPBS and incubated with secondary Alexa Fluor 488 or 647 conjugated antibodies (Invitrogen) in blocking solution (1:1,000) for 2 h. To label F-actin, fixed embryos were incubated with Phalloidin-Rhodamine (1:500, Molecular Probes), Phalloidin-Alexa Fluor 555 (1:500, Invitrogen) or SPY555-Actin (1:1,000, Spirochrome), and for nuclear staining with DAPI (Sigma) or Hoechst-33342 at 1:1,000.

### Image analysis

3D visualizations of embryos were performed using Imaris 9.7 software (Bitplane AG). The manual surface rendering module was used for cell segmentation. Cell volume was derived from the segmented data using the Imaris statistics module.

### Quantification of cell-cell contact

The cytoplasm of all cells in an embryo were segmented and converted into 3D meshes of approximately evenly spaced vertices. For each vertex in each cell the distance between it and the vertices of neighboring cells was calculated. If the distance between any two vertices on the surface of neighboring cells was below a threshold of 2 µm these points were considered to be in cell-cell contact. The percentage of points in cell-cell contact was then calculated for each cell.

### Analysis of direction of cytDNA production

cytDNA structures were segmented and the value of all pixels outside of these segmented regions set to 0. The apical-basal axis of each cell was approximated as a line that passes through the center of mass of the embryo and the center of mass of the cell's nucleus. A 2D slice along this axis was then taken through the embryo and rotated around the apical-basal axis by 180° in 1° increments to produce a stack of images. This stack of images was then collapsed into a single image producing a 'rotational maximum projection' of cytDNA structures in the perinuclear space. Images from multiple nuclei were then combined creating a heatmap of where cytDNA structures form with respect to the cells apical-basal axis.

### Statistical analysis

Statistical analyses were performed in Excel and GraphPad Prism. Data were analyzed for normality using a D'Agostino-Pearson omnibus normality test. Variables displaying a normal distribution were analyzed using an unpaired, two-tailed Student's t-test for two groups, and ANOVA with Dunnett's multiple comparisons test for more than two groups. Variables that did not follow a normal distribution were analyzed using an unpaired, two-tailed Mann-Whitney U-test for two groups, and Kruskal-Wallis test with Dunn's multiple comparisons test for more than two groups. Reproducibility was confirmed by independent experiments.

### Results

### Live imaging mouse embryos using fluorescent dyes

To establish the application of non-invasive imaging with subcellular resolution in live preimplantation embryos, we first tested the use of fluorescent dyes labeling specific cellular structures in the mouse embryo from the 2-cell to blastocyst stage (Fig. 1a and Fig. 4a). Incubation with the dye SPY650-DNA, which labels genomic DNA, and SPY555-actin, which labels F-actin, produced high signal to noise ratios (Fig. 4b). The labeling patterns were equivalent to those produced using microinjection of mRNAs coding for fluorescently-tagged histone 2B (H2B-GFP) and utrophin-RFP (Utr-RFP) (Fig. 1a-d), which have been extensively used for visualizing the dynamics of chromatin and F-actin in live embryos, respectively. The fluorescent dyes also produced images similar to fixed specimens stained with 4',6-diamidino-2-phenylindole (DAPI) and Phalloidin-Rhodamine (Fig. 4b).

Analysis of the live-imaging data confirmed that SPY650-DNA and SPY555-actin allowed us to perform a confocal three-dimensional (3D) scan of the embryo (at 5-10 minute intervals) and accurately visualize central events occurring during preimplantation development. This included: capturing the main phases of mitosis (Fig. 1b); visualizing the major changes in cell shape that characterize embryo compaction at the 8-cell stage (Fig. 1c; detecting cell polarization at the 8-cell stage identified by the formation of the apical domain, and establishment of apical F-actin rings at the 16-cell stage (Fig. 1d); tracking the expansion and zippering of F-actin rings, which seal the embryo prior to blastocyst formation (Fig. 1d); detecting the first internalized cells within the 16-cell embryo, which will form the ICM (Fig. 1e); and visualizing blastocyst expansion and hatching from the zona pellucida (Fig. 1f). Furthermore, embryos stained with these dyes produced offspring following transfer into pseudopregnant mice (Fig. 4c,d). Thus, the combination of fluorescent dyes with live-imaging permits the study of key events during preimplantation development, and can bypass cell-labeling approaches relying on mRNA or DNA microinjection.

### Cell dynamics underlying human preimplantation development

We next tested this approach to uncover cellular and morphogenetic processes underlying human development using embryos derived from *in vitro* fertilization (lVF). In addition to blastocysts, we obtained cleavage-stage embryos both pre- and post- compaction. We first studied cell cycle dynamics and captured the main phases of mitosis (Fig. 2a), which revealed that the duration of human mitosis was similar to those measured in mouse (Fig. 2b). By contrast, interphase was 24 ± 4% longer in human (16.1 ± 0.5 h versus 13.0 ± 0.5 h, P<0.0001) (Fig. 2b). This is consistent with previous reports showing a large conservation of the timing of mitosis across species, and studies proposing that the duration of morphogenetic processes in different species is determined predominantly by interphase.

The SPY650-FastAct dye also allowed us to capture the key changes in cell shape driving human embryo compaction that occur at the 16-cell stage (Fig. 2c). In line with studies in mouse, we measured an increase in cell-cell contact area and a decrease in cell sphericity, which serve as a proxy for compaction (Fig. 2d). Interestingly, mouse compaction occurs in a relatively synchronous manner across all cells of the embryo and lasts approximately 5 hours. By contrast, human embryo compaction occurs in a more asynchronous pattern over 12 hours (Fig. 2e). Furthermore, in the mouse embryo, compaction occurs at the 8-cell stage and is followed by the allocation of the first inner cells that will form the ICM during the 8- to 16-cell stage transition. This inner cell allocation is mediated by asymmetric cell divisions, triggered by asymmetric mitotic spindles, and apical constriction that helps to fully internalize these cells. In contrast, human compaction occurs at the 16-cell stage when the embryo already contains some cells occupying an inner position. Thus, inner-outer segregation and compaction occur concomitantly in the mouse and sequentially in the human embryo. Furthermore, in the mouse embryo SPY555-actin sharply labels the apical domains at the 8-cell stage and F-actin rings at the 16-cell stage (Fig. 1d). However, we did not detect the establishment of similar, well-defined apical structures in human. Instead, the human embryo maintained a more homogenous F-actin distribution across the apical cortex (Fig. 2f).

We then analyzed cell dynamics during blastocyst formation. Previous studies in human reported transient blastocoel collapses associated with low implantation potential, and work in mouse embryos proposed that cell divisions disrupt the trophectoderm layer causing these rapid reductions in blastocyst volume. By contrast, analysis of division patterns and blastocyst volume revealed that in the human blastocyst cell division patterns do not display high temporal synchronicity, neither do they correlate with blastocoel collapse (Fig. 2g). Thus, trophectoderm cells undergo divisions without overtly disrupting the cohesiveness of the trophectoderm layer. Furthermore, our imaging approach allowed us to visualize changes in cell morphology as the blastocyst undergoes hatching (Fig. 2h). Together, these results unveil cellular and morphogenetic processes underlying human preimplantation development and highlight differences between mouse and human.

### DNA loss from trophectoderm cell nuclei during blastocyst expansion

Live imaging using SPY650-DNA also allowed us to identify prominent cytoplasmic DNA structures appearing in the human blastocyst (Fig. 3a). We refer to these structures as cytDNA to distinguish them from mitochondrial and nuclear DNA. Preimplantation embryos often contain a larger number of aneuploid cells than somatic tissues. Whereas embryos in which all cells are aneuploid are thought to derive from chromosome segregation errors during meiosis, mosaic aneuploid patterns have been proposed to result from segregation errors during mitosis. Our imaging approach enabled us to follow mitoses and identify lagging chromosomes producing micronuclei, occurring in 4.2% of tracked cell divisions (Fig. 5a-c). However, the resulting micronuclei appeared morphologically distinct from cytDNA (Fig. 5c,d). Furthermore, while micronuclei are typically enclosed by a nuclear envelope-like structure, most cytDNA did not display Lamin staining around them (Fig. 5d). This suggests that cytDNA structures may not derive from chromosome segregation errors during mitosis.

Visualization of nuclear morphology revealed bud-like structures labeled by SPY650-DNA protruding outwards from the nucleus (Fig. 3b). Importantly, tracking these nuclear buds over time showed that they are lost into the cytoplasm, producing cytDNA (Fig. 3b). These events showing loss of DNA from the nucleus could be observed in advanced-stage but not early-cavitating blastocysts, in both human and mouse embryos (Fig. 3a-d, Fig. 6a-c). Over 15 hours, we tracked the generation of 2-5 cytDNA structures within trophectoderm cells per blastocyst, representing 2.9% ± 0.3% of trophectoderm cells imaged (Fig. 3c, Fig. 6c). In some cases, the same cell nucleus underwent more than one DNA loss event producing cytDNA (Fig. 6d). Moreover, the majority of tracked cells containing cytDNA subsequently underwent mitosis (Fig. 6e). Computational segmentation of cell nuclei, nuclear buds and cytDNA structures demonstrated that nuclear volume in cells producing cytDNA had decreased following DNA release compared to cells without cytDNA, whose nuclear volumes remained stable (Fig 3d, Fig. 6b). Human and mouse embryos stained with DAPI, Hoechst-33342, or an antibody recognizing double stranded DNA confirmed the presence of similar nuclear buds and cytDNA structures (Fig. 3e,f, Fig. 6f). We also validated these findings in embryos produced from both superovulated and naturally mated mice, and embryos both cultured or freshly flushed, indicating no overt effects from ovarian stimulation or culture conditions (Fig. 6g).

We next explored whether cytDNA structures are associated with programmed cell death (PCD), which is typically characterized by nuclear DNA fragments. These pyknotic fragments are marked by cleaved caspase-3 and gradually cleared from apoptotic cells. Immunofluorescence in mouse embryos showed cleaved caspase-3 staining surrounding cytDNA (Fig. 7a,b). However, the nuclei of most cells containing cytDNA remained intact and did not display a pyknotic morphology or cleaved Caspase-3 staining (Fig. 7a,b). To further track the fate of these cells over time, we used an active-caspase fluorescent reporter (CellEvent Caspase-3/7 Green). This demonstrated that cells containing caspase-labeled cytDNA can subsequently undergo mitosis (Fig. 7c,d). Together, these results show that cytDNA structures are not generated during chromosome segregation errors during mitosis or produced by cells undergoing stereotypical PCD. Instead, they originate from intact nuclei and can persist within the cytoplasm and be inherited following cell division.

We finally investigated processes that could lead to the loss of DNA from cell nuclei *in vivo.* Recent work showed that when cultured cells migrate through mechanically-confined microenvironments their nuclei deform causing transient ruptures. Interestingly, cells in the embryo also become mechanically constrained when the blastocoel expands and the trophectoderm layer flattens along its apical-basal axis. Consistent with this, trophectoderm cell nuclei adopt a flatter morphology orthogonal to their apical-basal axis during embryo cavitation. Moreover, nuclear buds are also detected predominantly during cavity expansion and localize along the same orthogonal axis (Fig. 3d,g; Fig. 6b,h). By contrast, cell nuclei within the ICM do not undergo such pronounced morphological deformation during blastocoel expansion (Fig. 6h) and moreover, cytDNA is detected at a higher frequency in the trophectoderm (Fig. 6f).

To further understand why a subset of trophectoderm cell nuclei are susceptible to DNA loss during blastocyst expansion, we analyzed the organization of the keratin filament network. Keratins confer cellular stability in various tissues experiencing mechanical stress and can mechanically support cell nuclei in various systems. Furthermore, we previously showed that keratins stabilize the apical cortex of the trophectoderm. Analysis of human embryos revealed that, in addition to the cortical keratin network, keratins establish a prominent cage-like network surrounding most trophectoderm nuclei (Fig. 3h). However, keratin expression is heterogeneous throughout the trophectoderm, and we found that cells with lower keratin levels contain more cytDNA structures (Fig. 3h). Moreover, using siRNAs for K8 and K18 microinjected into one cell of the 2-cell stage disrupts the keratin network and causes an increase in cytDNA structures (Fig. 3i). Thus, this indicates that trophectoderm cells with lower keratin expression are more prone to undergo DNA loss producing cytDNA.

### Conclusions

The main events driving preimplantation development have thus far been inferred form work in the mouse embryo. Here, the use of fluorescent dyes and live-imaging allowed us to reveal cellular and morphogenetic processes as they occur in real time within the preimplantation human embryo. The data expose several differences between human and mouse development. While the duration of mitosis is relatively conserved, interphase is longer in human. Furthermore, human embryo compaction does not precede the internalization of the first cells that will form the ICM. Instead, compaction and inner-cell segregation take place concomitantly. Moreover, cell division patterns do not correlate with collapses in human blastocyst volume. Instead, the trophectoderm can maintain a cohesive tissue organization during cavity expansion, even with cells containing cytDNA.

Our identification of cytDNA structures show that some cell nuclei lose DNA into the cytoplasm in both human and mouse blastocysts, as trophectoderm cells become mechanically constrained during cavity expansion. Moreover, while most trophectoderm nuclei are surrounded by a keratin filament network, cells with lower keratin expression or microinjected with K8+K18 siRNAs display more cytDNA structures. This indicates that the perinuclear network of keratin filaments may mechanically protect the nucleus against DNA loss.

Our results open the question of whether cytDNA influences specific cell functions or fate. We found that cytDNA is detected by caspases, yet these cells do not undergo stereotypical PCD. Instead, cytDNA can persist within the cytoplasm and even be inherited following cell division. Thus, cytDNA could recruit caspases to trigger non-apoptotic signaling, as found during caspase-dependent cell shape changes in *Drosophila.* It remains technically unfeasible to isolate and distinguish specific DNA sequences in cytDNA and micronuclei. This would require imaging DNA loss and chromosome segregation errors in real time, followed by sequencing each subcellular structure (i.e., nucleus, cytDNA and micronuclei) in single cells. However, our results showing a reduction in nuclear volume following DNA loss suggests that the resulting nuclei have an abnormal genomic complement. Therefore, we propose that the loss of DNA from the interphase nucleus could represent an additional process accounting for mosaic aneuploidy detected in trophectoderm biopsies, thus far predominantly linked to chromosome segregation errors in mitosis and cell death. Finally, as the imaging approach established here can bypass the requirement for microinjection or genetic manipulation to fluorescently label cells, it may be feasible to apply it to non-invasively monitor some of these processes during assisted reproduction.

## Claims

1. An *in* vitro method of obtaining one or more fluorescent images of one or more isolated cells, the method comprising the steps of:
(a) incubating the one or more isolated cells with a first probe for fluorescence imaging of nuclear DNA, wherein the first probe comprises a molecule capable of binding to nuclear DNA conjugated to a first fluorophore, and wherein the incubation produces a stain indicative of the presence of nuclear DNA,
(b) prior to, simultaneously to, or subsequently to step (a), incubating the one or more isolated cells with a second probe for fluorescence imaging of actin, wherein the second probe comprises a molecule capable of binding to actin conjugated to a second fluorophore, and wherein the incubation produces a stain indicative of the presence of actin, and
(c) providing one or more images of the one or more isolated cells that have been stained with both the first and the second probe, said one or more images being obtained by fluorescence microscopy,
wherein the first and second probes are cell permeable, wherein one of the fluorophores has a maximum absorbance wavelength (A_{λ}) of 652 nm and a maximum fluorescence wavelength (F_{λ}) of 674 nm and the other fluorophore has a maximum absorbance wavelength (A_{λ}) of 555 nm and a maximum fluorescence wavelength (F_{λ}) of 580 nm, and wherein the in vitro method does not involve any damage or destruction of the one or more cells to which the method is applied.

2. The method according to claim 2, wherein the probe for fluorescence imaging of actin specifically labels F-actin.

3. The method according to any of claims 1 or 2, wherein the molecule capable of binding to nuclear DNA is a Bisbenzimide or a Hoechst stain.

4. The method according to any of claims 1 to 3, wherein the molecule capable of binding to actin is a Jasplakinolide.

5. The method according to any of claims 1 to 4, wherein the one or more isolated cells are comprised in a mammalian embryo, wherein said mammalian embryo is cultured under conditions to promote survival, growth and/or development, and wherein the one or more images provided in step (c) are analysed to determine whether the embryo presents developmental abnormalities as compared to control embryos.

6. The method according to any of claims 1 to 5, wherein the one or more isolated cells are comprised in a mammalian embryo, wherein said mammalian embryo is cultured under conditions to promote survival, growth and/or development, and wherein the method comprises a step (d) of visualizing on the one or more images provided by fluorescence microscopy the
labelled nuclear DNA of the one or more cells and determining the presence or absence of nuclear DNA loss events in said cells, said events being **characterized by** the presence of labelled nuclear DNA protruding outwards from the nucleus into the cytoplasm of said one or more cells.

7. The method according to claim 6, wherein step (c) is performed for two or more time intervals thereby providing time-lapse images of the mammalian embryo, and wherein said time intervals are performed during the morphological events in the embryo development that take place when embryo is in blastocyst stage, preferably when the trophectoderm layer becomes constrained during cavity expansion in the blastocyst.

8. The method according to any of claims 6 or 7, wherein the mammalian embryo is diagnosed with aneuploidy if nuclear DNA loss events are determined in at least one of its cells comprised in the embryo.

9. The method according to any of claims 6 to 8, wherein the mammalian embryo is a human embryo.

10. In vitro use of a first probe in combination with a second probe for obtaining a fluorescent image of one or more isolated cells, wherein the first probe comprises a molecule capable of binding to nuclear DNA conjugated to a first fluorophore, wherein the second probe comprises a molecule capable of binding to actin conjugated to a second fluorophore, wherein one of the fluorophores has a maximum absorbance wavelength (A_{λ}) of 652 nm and a maximum fluorescence wavelength (F_{λ}) of 674 nm and the other fluorophore has a maximum absorbance wavelength (A_{λ}) of 555 nm and a maximum fluorescence wavelength (F_{λ}) of 580 nm.

11. In vitro use of a first probe in combination with a second probe for determining whether one or more cells that are undergoing cell division are losing or will lose part of their nuclear DNA, wherein the first probe comprises a molecule capable of binding to nuclear DNA conjugated to a first fluorophore, wherein the second probe comprises a molecule capable of binding to actin conjugated to a second fluorophore, wherein one of the fluorophores has a maximum absorbance wavelength (A_{λ}) of 652 nm and a maximum fluorescence wavelength (F_{λ}) of 674 nm and the other fluorophore has a maximum absorbance wavelength (A_{λ}) of 555 nm and a maximum fluorescence wavelength (F_{λ}) of 580 nm.

12. The in vitro uses according to any of claims 10 or 11, wherein the molecule capable of binding to nuclear DNA is a Bisbenzimide or a Hoechst stain.

13. The in vitro uses according to any of claims 10 to 12, wherein the molecule capable of binding to actin is a Jasplakinolide.

14. A computer implemented method for carrying out the method or the uses of any of claims 1 to 13.

15. A computer program product comprising instructions such that when processed by a processor, configures it to carry out the method or the uses of any of claims 1 to 14.

16. A kit suitable for obtaining one or more fluorescent images of one or more isolated cells, the kit comprising a first and a second probe, wherein the first probe comprises a molecule capable of binding to nuclear DNA conjugated to a first fluorophore, wherein the second probe comprises a molecule capable of binding to actin conjugated to a second fluorophore, and wherein one of the fluorophores has a maximum absorbance wavelength (A_{λ}) of 652 nm and a maximum fluorescence wavelength (F_{λ}) of 674 nm and the other fluorophore has a maximum absorbance wavelength (A_{λ}) of 555 nm and a maximum fluorescence wavelength (F_{λ}) of 580 nm.
